(19) Europäisches Patentamt
European Patent Office
Office européen des brevets

(11) EP 2 604 698 A1

(12) EUROPEAN PATENT APPLICATION
published in accordance with Art. 153(4) EPC

(43) Date of publication:
**19.06.2013 Bulletin 2013/25**

(21) Application number: **11816408.6**

(22) Date of filing: **09.08.2011**

(51) Int Cl.:
***C12Q 1/26*** (2006.01) ***C12Q 1/28*** (2006.01)
***C12Q 1/37*** (2006.01)

(86) International application number:
**PCT/JP2011/068103**

(87) International publication number:
**WO 2012/020744 (16.02.2012 Gazette 2012/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.08.2010 JP 2010180562**

(71) Applicant: **Kyowa Medex CO., LTD.**
**Tokyo 104-6004 (JP)**

(72) Inventors:
- **MURAKAMI, Tomomi**
  **Shizuoka 411-0932 (JP)**
- **SOYA, Haruyo**
  **Shizuoka 411-0932 (JP)**
- **OHSUGI, Yu**
  **Shizuoka 411-0932 (JP)**
- **YODA, Ayako**
  **Shizuoka 411-0932 (JP)**
- **TSUNODA, Haruki**
  **Shizuoka 411-0932 (JP)**
- **MATSUSHITA, Masashi**
  **Shizuoka 411-0932 (JP)**

(74) Representative: **Vossius & Partner**
**Siebertstrasse 4**
**81675 München (DE)**

## (54) METHOD FOR MEASURING GLYCATED HEMOGLOBIN

(57) It is to provide a method for measuring glycated hemoglobin in a hemoglobin-containing sample, comprising reacting the hemoglobin-containing sample with a proteolytic enzyme in the presence of a surfactant, and then reacting the obtained reaction product with fructosyl peptide oxidase, wherein at least one of the former reaction and the latter reaction is performed in the presence of an isothiazolinone derivative; and measuring the generated hydrogen peroxide. The present invention provides a method for accurately and highly sensitively measuring glycated hemoglobin in a hemoglobin-containing sample without being influenced by hemoglobin.



Printed by Jouve, 75001 PARIS (FR)

## Description

### Technical Field

The present invention relates to a method, a reagent, and a kit for measuring glycated hemoglobin.

### Background Art

Glycated hemoglobin is a glycation product of hemoglobin in which glucose is bound thereto. Hemoglobin takes a tetrameric structure consisting of α and β chains. The glycated product of N terminus of β chain of hemoglobin is called hemoglobin Alc, which increases with increase in blood glucose level and as such, is measured as a diabetes mellitus marker in clinical laboratory examinations.

Known methods for measuring glycated hemoglobin include, for example, chromatography such as HPLC, electrophoresis, antibody-based immunoassay such as latex immunoagglutination assay, and enzymatic assay using an enzyme reactive to a glycated protein and an enzyme reactive to a glycated peptide and/or a glycated amino acid.

A known method for enzymatically measuring glycated hemoglobin (absorptiometry) comprises: first denaturing hemoglobin in a hemoglobin-containing sample using a denaturant; reacting the denatured hemoglobin with a proteolytic enzyme; subsequently reacting the generated glycated peptide with glycated peptide oxidase; reacting the generated hydrogen peroxide with a chromogen capable of developing color by oxidation in the presence of a peroxidatively active substance such as peroxidase to convert the chromogen to a dye; and measuring the glycated hemoglobin on the basis of the absorbance of the generated dye.

This measurement of glycated hemoglobin based on absorptiometry is disadvantageously susceptible to interference with hemoglobin present in large amounts in the sample. For example, a method using a cationic surfactant and/or an amphoteric surfactant (Patent Document 1), a method using a sulfone compound and/or a nitro compound (Patent Documents 2 and 3), a method using a tetrazolium compound (Patent Document 4), and a method using a particular anionic surfactant such as polyoxyethylene alkyl ether sulfates (Patent Document 5) are known as solutions to this problem.

Unfortunately, these methods cannot always avoid the influence of hemoglobin. There is a demand for a method for accurately measuring glycated hemoglobin in a hemoglobin-containing sample without being influenced by hemoglobin.

### Prior Art Documents

### Patent Documents

Patent Document 1: Japanese unexamined Patent Application Publication No. 3-010696
Patent Document 2: WO2003/10701
Patent Document 3: Japanese unexamined Patent Application Publication No. 2007-147630
Patent Document 4: Japanese unexamined Patent Application Publication No. 2000-210100
Patent Document 5: WO2005/04985

### Summary of the Invention

### Problems to be Solved by the Invention

An object of the present invention is to provide a method and a reagent for accurately and highly sensitively measuring glycated hemoglobin in a hemoglobin-containing sample without being influenced by hemoglobin.

### Means to Solve the Problems

As a result of conducting diligent studies, the present inventors have found that a method comprises reacting a hemoglobin-containing sample with a proteolytic enzyme in the presence of a surfactant and then reacting the obtained reaction product with fructosyl peptide oxidase, wherein at least one of the former reaction and the latter reaction is performed in the presence of an isothiazolinone derivative; and measuring the generated hydrogen peroxide, whereby glycated hemoglobin in the hemoglobin-containing sample can be measured accurately and highly sensitively without being influenced by hemoglobin. On the basis of these findings, the present invention has been completed. Specifically,

the present invention relates to the following [1] to [15]:

[1] A method for measuring glycated hemoglobin in a hemoglobin-containing sample, comprising reacting the hemoglobin-containing sample with a proteolytic enzyme in the presence of a surfactant, and then reacting the obtained reaction product with fructosyl peptide oxidase, wherein at least one of the former reaction and the latter reaction is performed in the presence of an isothiazolinone derivative; and measuring the generated hydrogen peroxide.

[2] The method according to [1], wherein the isothiazolinone derivative is a compound represented by the following formula (I):

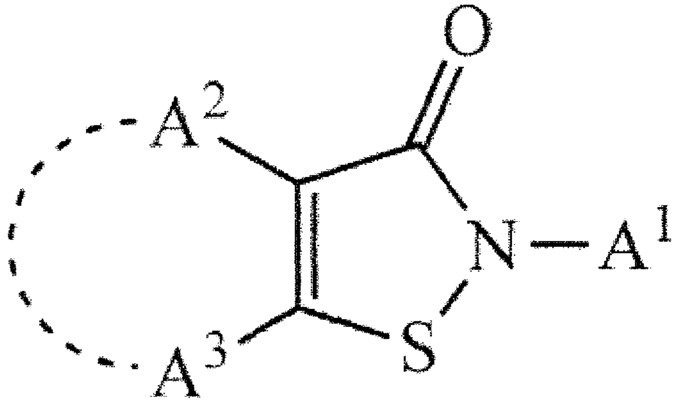

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

[3] The method according to [2], wherein the compound represented by the formula (I) is an isothiazolinone derivative selected from the group consisting of 2-alkyl-4-isothiazolin-3-one, 1,2-benzisothiazol-3(2H)-one, and 2-alkyl-4,5-dihalogeno-4-isothiazolin-3-one.

[4] The method according to any one of [1] to [3], wherein the measurement of the hydrogen peroxide is performed using a hydrogen peroxide measuring reagent.

[5] The method according to [4], wherein the hydrogen peroxide measuring reagent is a reagent comprising peroxidase and a leuco chromogen.

[6] A reagent for measuring glycated hemoglobin in a hemoglobin-containing sample, comprising a proteolytic enzyme, fructosyl peptide oxidase, an isothiazolinone derivative, and a surfactant.

[7] The reagent according to [6], wherein the isothiazolinone derivative is a compound represented by the following formula (I):

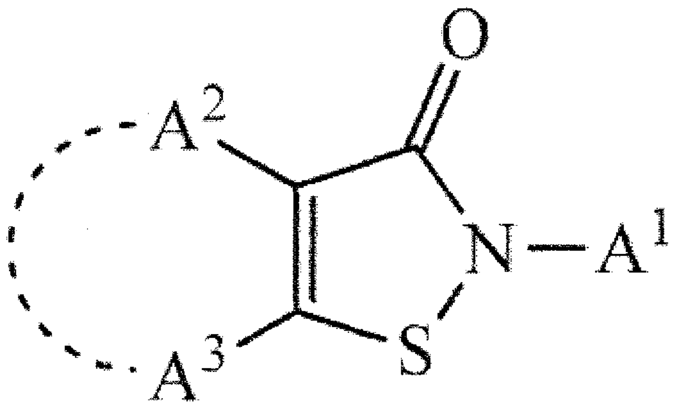

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

[8] The reagent according to [7], wherein the compound represented by the formula (I) is an isothiazolinone derivative selected from the group consisting of 2-alkyl-4-isothiazolin-3-one, 1,2-benzisothiazol-3(2H)-one, and 2-alkyl-4,5-dihalogeno-4-isothiazolin-3-one.

[9] The reagent according to any one of [6] to [8], further comprising a hydrogen peroxide measuring reagent.

[10] The reagent according to [9], wherein the hydrogen peroxide measuring reagent is a reagent comprising peroxidase and a leuco chromogen.

[11] A kit for measuring glycated hemoglobin in a hemoglobin-containing sample comprising: a first reagent comprising a proteolytic enzyme, an isothiazolinone derivative, and a surfactant; and a second reagent comprising fructosyl peptide oxidase.

[12] A kit for measuring glycated hemoglobin in a hemoglobin-containing sample comprising: a first reagent comprising a proteolytic enzyme and a surfactant; and a second reagent comprising fructosyl peptide oxidase and an isothiazolinone derivative.

[13] The kit according to [11] or [12], wherein the isothiazolinone derivative is a compound represented by the following formula (I):

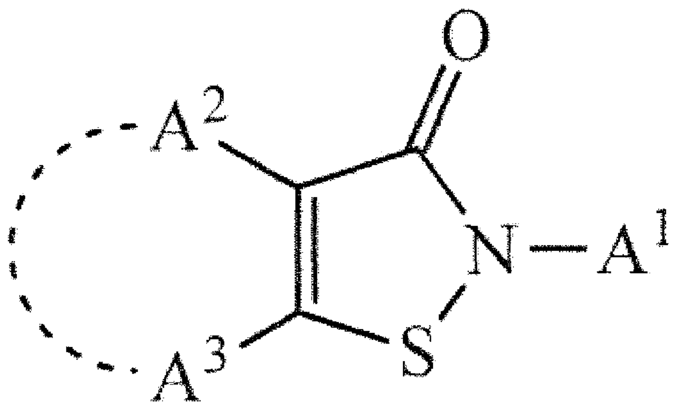

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

[14] The kit according to [13], wherein the compound represented by the formula (I) is an isothiazolinone derivative selected from the group consisting of 2-alkyl-4-isothiazolin-3-one, 1,2-benzisothiazol-3(2H)-one, and 2-alkyl-4,5-dihalogeno-4-isothiazolin-3-one.

[15] The kit according to any one of [11] to [14], wherein the first reagent and the second reagent or the second reagent and the first reagent further comprise peroxidase and a leuco chromogen, respectively.

**Effect of the Invention**

The present invention provides a method, a reagent, and a kit for accurately and highly sensitively measuring glycated hemoglobin in a hemoglobin-containing sample without being influenced by hemoglobin.

**Brief Description of Drawings**

[Figure 1] Figure 1 is a graph showing the relationship between hemoglobin concentration and reaction absorbance in the measurement of hemoglobin Alc (hereinafter, also referred to as HbA1c) in a specimen using kits of Example 1, Example 2, and Comparative Example 1. The symbol ● represents the results of measurement using the kit of Comparative Example 1. The symbol Δ represents the results of measurement using the kit of Example 1. The symbol ■ represents the results of measurement using the kit of Example 2. The ordinate represents reaction absorbance ($\times 10^{-4}$ Abs). The abscissa represents hemoglobin concentration (mg/mL).

[Figure 2] Figure 2 is a graph showing the relationship between hemoglobin concentration and reaction absorbance in the measurement of HbA1c in a specimen using kits of Example 9, Example 10, and Comparative Example 5. The symbol ● represents the results of measurement using the kit of Comparative Example 5. The symbol ▲ represents the results of measurement using the kit of Example 9. The symbol □ represents the results of measurement using the kit of Example 10. The ordinate represents reaction absorbance ($\times 10^{-4}$ Abs). The abscissa represents hemoglobin concentration (mg/mL).

[Figure 3] Figure 3 is a graph showing the relationship between hemoglobin concentration and reaction absorbance

in the measurement of HbA1c in a specimen using kits of Example 7, Example 8, and Comparative Example 4. The symbol ● represents the results of measurement using the kit of Comparative Example 4. The symbol Δ represents the results of measurement using the kit of Example 7. The symbol ■ represents the results of measurement using the kit of Example 8. The ordinate represents reaction absorbance ($\times 10^{-4}$ Abs). The abscissa represents hemoglobin concentration (mg/mL).
[Figure 4] Figure 4 is a graph showing the relationship between hemoglobin concentration and reaction absorbance in the measurement of HbA1c in a specimen using kits of Example 15, Example 16, and Comparative Example 8. The symbol ● represents the results of measurement using the kit of Comparative Example 8. The symbol ▲ represents the results of measurement using the kit of Example 15. The symbol ■ represents the results of measurement using the kit of Example 16. The ordinate represents reaction absorbance ($\times 10^{-4}$ Abs). The abscissa represents hemoglobin concentration (mg/mL).
[Figure 5] Figure 5 is a graph showing the relationship between hemoglobin concentration and reaction absorbance in the measurement of HbA1c in a specimen using kits of Example 21, Example 22, and Comparative Example 11. The symbol ● represents the results of measurement using the kit of Comparative Example 11. The symbol ■ represents the results of measurement using the kit of Example 21. The symbol ▲ represents the results of measurement using the kit of Example 22. The ordinate represents reaction absorbance ($\times 10^{-4}$ Abs). The abscissa represents hemoglobin concentration (mg/mL).

## Mode for Carrying Out the Invention

(1) Method for measuring glycated hemoglobin in hemoglobin-containing sample

The method for measuring glycated hemoglobin in a hemoglobin-containing sample according to the present invention comprises: reacting glycated hemoglobin in the hemoglobin-containing sample with a proteolytic enzyme in the presence of a surfactant and then reacting the obtained reaction product with fructosyl peptide oxidase, wherein at least one of the former reaction and the latter reaction is performed in the presence of an isothiazolinone derivative; and measuring the generated hydrogen peroxide. The isothiazolinone derivative can be allowed to exist in the reaction of fructosyl peptide oxidase or can be allowed to exist in both of the reaction of a proteolytic enzyme and the reaction of fructosyl peptide oxidase.
Specifically, the measuring method comprises the following steps:

<Measuring Method 1>

(1) a step of reacting glycated hemoglobin in the hemoglobin-containing sample with a proteolytic enzyme in the presence of a surfactant;
(2) a step of reacting the reaction product obtained in step (1) with fructosyl peptide oxidase in the presence of an isothiazolinone derivative to generate hydrogen peroxide;
(3) a step of measuring the hydrogen peroxide generated in step (2); and
(4) a step of determining the concentration of glycated hemoglobin in the hemoglobin-containing sample from the amount of the hydrogen peroxide measured in step (3) on the basis of a calibration curve representing the relationship between the amount of hydrogen peroxide and the concentration of glycated hemoglobin, prepared in advance using known concentrations of glycated hemoglobin.

<Measuring Method 2>

(1) a step of reacting glycated hemoglobin in the hemoglobin-containing sample with a proteolytic enzyme in the presence of an isothiazolinone derivative and a surfactant;
(2) a step of reacting the reaction product obtained in step (1) with fructosyl peptide oxidase to generate hydrogen peroxide;
(3) a step of measuring the hydrogen peroxide generated in step (2); and
(4) a step of determining the concentration of glycated hemoglobin in the hemoglobin-containing sample from the amount of the hydrogen peroxide measured in step (3) on the basis of a calibration curve representing the relationship between the amount of hydrogen peroxide and the concentration of glycated hemoglobin, prepared in advance using known concentrations of glycated hemoglobin.

The method for measuring glycated hemoglobin in a hemoglobin-containing sample according to the present invention also encompasses even a method comprising calculating the ratio of the amount of glycated hemoglobin to the amount of total hemoglobin (i.e., total hemoglobin composed of hemoglobin and glycated hemoglobin) in the hemoglobin-containing sample. In this case, a method for measuring glycated hemoglobin in a hemoglobin-containing sample according to the present invention specifically comprises the following steps:

<Measuring Method 3>

(1) a step of determining the amount of total hemoglobin (i.e., total hemoglobin composed of hemoglobin and glycated hemoglobin) in the hemoglobin-containing sample;
(2) a step of reacting glycated hemoglobin in the hemoglobin-containing sample with a proteolytic enzyme in the presence of a surfactant;
(3) a step of reacting the reaction product obtained in step (2) with fructosyl peptide oxidase in the presence of an isothiazolinone derivative to generate hydrogen peroxide;
(4) a step of measuring the hydrogen peroxide generated in step (3);
(5) a step of determining the amount of glycated hemoglobin in the hemoglobin-containing sample from the amount of the hydrogen peroxide measured in step (4) on the basis of a calibration curve representing the relationship between the amount of hydrogen peroxide and the amount of glycated hemoglobin, prepared in advance using known amounts of glycated hemoglobin; and
(6) a step of calculating the ratio of the amount of glycated hemoglobin to the amount of total hemoglobin in the hemoglobin-containing sample from the amount of total hemoglobin determined in step (1) and the amount of glycated hemoglobin determined in step (5).

Step (1) of determining the amount of total hemoglobin can be performed after step (2).

<Measuring Method 4>

(1) a step of determining the amount of total hemoglobin (i.e., total hemoglobin composed of hemoglobin and glycated hemoglobin) in the hemoglobin-containing sample;
(2) a step of reacting glycated hemoglobin in the hemoglobin-containing sample with a proteolytic enzyme in the presence of an isothiazolinone derivative and a surfactant;
(3) a step of reacting the reaction product obtained in step (2) with fructosyl peptide oxidase to generate hydrogen peroxide;
(4) a step of measuring the hydrogen peroxide generated in step (3);
(5) a step of determining the amount of glycated hemoglobin in the hemoglobin-containing sample from the amount of the hydrogen peroxide measured in step (4) on the basis of a calibration curve representing the relationship between the amount of hydrogen peroxide and the amount of glycated hemoglobin, prepared in advance using known amounts of glycated hemoglobin; and
(6) a step of calculating the ratio of the amount of glycated hemoglobin to the amount of total hemoglobin in the hemoglobin-containing sample from the amount of total hemoglobin determined in step (1) and the amount of glycated hemoglobin determined in step (5).

Step (1) of determining the amount of total hemoglobin can be performed after step (2).

The hemoglobin-containing sample used in the measuring method of the present invention is not particularly limited as long as the sample contains hemoglobin and is applicable to the method for measuring glycated hemoglobin according to the present invention. Examples thereof include whole blood, blood cells, mixed samples of blood cells and plasma, and hemolyzed samples of these samples. The hemolyzing treatment is not particularly limited as long as the treatment hemolyzes whole blood, blood cells, or mixed samples of blood cells and plasma. Examples thereof include physical, chemical, and biological methods. Examples of the physical method include a method using a hypotonic solution such as distilled water, and a method using sonic waves. Examples of the chemical method include a method using an organic solvent such as methanol, ethanol, or acetone, and a method using a polyoxyethylene surfactant. Examples of the biological method include a method using an antibody or a complement.

The glycated hemoglobin according to the present invention is generated by binding of a sugar such as glucose to hemoglobin. Examples thereof include hemoglobin Ala, hemoglobin Alb, and hemoglobin A1c. Hemoglobin A1c is

preferable.

The isothiazolinone derivative according to the present invention is not particularly limited as long as the isothiazolinone derivative enables the method for measuring glycated hemoglobin according to the present invention. Examples thereof include a compound represented by the following formula (I) [hereinafter, referred to as compound (I)]:

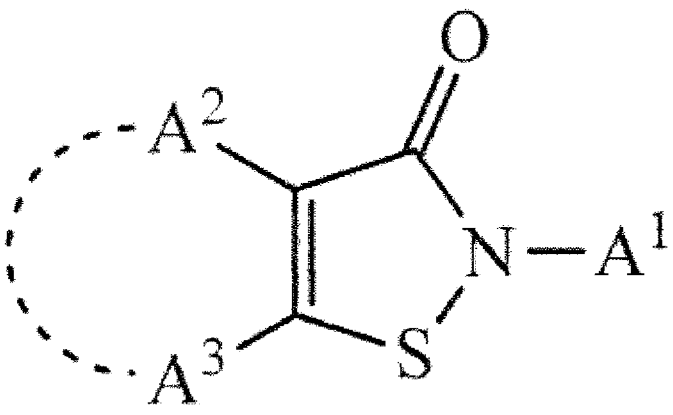

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

In compound (I), $A^1$ represents substituted or unsubstituted alkyl; and $A^2$ and $A^3$ are the same or different and each represent a hydrogen atom, a substituted or unsubstituted alkyl, or a halogen atom, or $A^2$ and $A^3$ together form a ring structure. Examples of alkyl in the substituted or unsubstituted alkyl include linear alkyl having 1 to 20 carbon atoms and branched alkyl having 3 to 20 carbon atoms. Examples of the linear alkyl having 1 to 20 carbon atoms include methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 3 to 20 carbon atoms include isopropyl, isobutyl, isopentyl, isohexyl, isoheptyl, isooctyl, isononyl, isodecyl, isoundecyl, isododecyl, isotridecyl, isotetradecyl, isopentadecyl, isohexadecyl, isoheptadecyl, isooctadecyl, isononadecyl, isoicosyl, and octyldodecyl. Examples of the halogen atom include chlorine, bromine, and iodine atoms. Examples of the substituent in the substituted alkyl include a phenyl group, a hydroxy group, a sulfo group, a cyano group, and a halogen atom. Examples of the halogen atom include the aforementioned halogen atom.

Examples of the ring structure formed by $A^2$ and $A^3$ together include benzene and naphthalene rings.

Specific examples of compound (I) include 2-alkyl-4-isothiazolin-3-one, 1,2-benzisothiazol-3(2H)-one, and 2-alkyl-4,5-dihalogeno-9-isothiazolin-3-one. Examples of commercially available compound (I) include 2-octyl-4-isothiazolin-3-one (manufactured by Tokyo Chemical Industry Co., Ltd.), 1,2-benzisothiazol-3(2H)-one (manufactured by Wako Pure Chemical Industries, Ltd.), and 4,5-dichlore-2-octyl-4-isothiazolin-3-one (manufactured by HighChem Co., Ltd.).

In the method for measuring glycated hemoglobin according to the present invention, the concentration of the isothiazolinone derivative in the reaction solution is not particularly limited as long as the concentration enables the method for measuring glycated hemoglobin according to the present invention. The concentration is usually 0.005 to 20 mmol/L, preferably 0.01 to 10 mmol/L.

The surfactant according to the present invention is not particularly limited as long as the surfactant enables the method for measuring glycated hemoglobin according to the present invention. Examples thereof include cationic surfactants, anionic surfactants, amphoteric surfactants, and nonionic surfactants.

Examples of the cationic surfactants include quaternary ammonium salts, pyridinium salts, phosphonium salts, imidazolium salts, and isoquinolinium salts. A quaternary ammonium salt, a pyridinium salt, or a phosphonium salt is preferable.

The quaternary ammonium salt is preferably a quaternary ammonium salt having at least one linear alkyl having 8 to 20 carbon atoms. Examples of the linear alkyl having 8 to 20 carbon atoms include octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl.

Specific examples (products) of the quaternary ammonium salt include decyl trimethyl ammonium chloride, decyl trimethyl ammonium bromide (hereinafter, referred to as C10TMA), dodecyl trimethyl ammonium chloride, dodecyl trimethyl ammonium bromide, hexadecyl trimethyl ammonium chloride, hexadecyl trimethyl ammonium bromide, didecyl dimethyl ammonium chloride, didecyl dimethyl ammonium bromide, didodecyl dimethyl ammonium chloride, and didodecyl dimethyl ammonium bromide (all manufactured by Tokyo Chemical Industry Co., Ltd.).

A pyridinium salt represented by the following general formula (II) [hereinafter, referred to as compound (II)] is

used as the pyridinium salt:

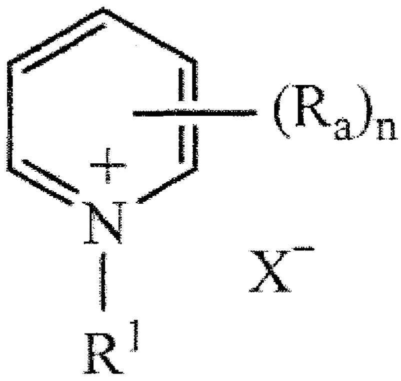

(II)

wherein $R^1$ represents substituted or unsubstituted alkyl or substituted or unsubstituted alkenyl; $R_a$ represents a hydrogen atom, substituted or unsubstituted alkyl, or substituted or unsubstituted alkenyl; n represents an integer of 1 to 5; and $X^-$ represents a monovalent anion.

Examples of alkyl in the substituted or unsubstituted alkyl represented by $R^1$ include linear alkyl having 1 to 20 carbon atoms, and branched alkyl having 3 to 20 carbon atoms. Linear alkyl having 8 to 20 carbon atoms, or branched alkyl having 8 to 20 carbon atoms is preferable. Examples of the linear alkyl having 1 to 20 carbon atoms include methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 3 to 20 carbon atoms include isopropyl, isobutyl, isopentyl, isohexyl, isoheptyl, isooctyl, isononyl, isodecyl, isoundecyl, isododecyl, isotridecyl, isotetradecyl, isopentadecyl, isohexadecyl, isoheptadecyl, isooctadecyl, isononadecyl, isoicosyl, and octyldodecyl. Examples of the linear alkyl having 8 to 20 carbon atoms include the aforementioned linear alkyl having 8 to 20 carbon atoms. Examples of the branched alkyl having 8 to 20 carbon atoms include isooctyl, isononyl, isodecyl, isoundecyl, isododecyl, isotridecyl, isotetradecyl, isopentadecyl, isohexadecyl, isoheptadecyl, isooctadecyl, isanorxadecyl, isoicosyl, and octyldodecyl.

Examples of alkenyl in the substituted or unsubstituted alkenyl represented by $R^1$ include alkenyl having 2 to 20 carbon atoms. Alkenyl having 8 to 20 carbon atoms is preferable. Examples of the alkenyl having 2 to 20 carbon atoms include vinyl, propenyl, allyl, butenyl, pentenyl, hexenyl, heptenyl, octenyl, nonenyl, decenyl, undecenyl, dodecenyl, tetradecenyl, pentadecenyl, hexadecenyl, heptadecenyl, octadecenyl, oleyl, nonadecenyl, and icosenyl. Examples of the alkenyl having 8 to 20 carbon atoms include octenyl, nonenyl, decenyl, undecenyl, dodecenyl, tetradecenyl, pentadecenyl, hexadecenyl, heptadecenyl, octadecenyl, oleyl, nonadecenyl, and icosenyl.

Examples of the substituent in the substituted alkyl or the substituted alkenyl represented by $R^1$ include a phenyl group, a hydroxy group, a sulfo group, a cyano group, and halogen atoms. Examples of the halogen atoms include chlorine, bromine, and iodine atoms.

Examples of alkyl in the substituted or unsubstituted alkyl represented by $R_a$ include linear alkyl having 1 to 20 carbon atoms, and branched alkyl having 3 to 20 carbon atoms. Examples of the linear alkyl having 1 to 20 carbon atoms include the aforementioned linear alkyl having 1 to 20 carbon atoms. Examples of the branched alkyl having 3 to 20 carbon atoms include the aforementioned branched alkyl having 3 to 20 carbon atoms.

Examples of alkenyl in the substituted or unsubstituted alkenyl represented by $R_a$ include alkenyl having 2 to 20 carbon atoms. Examples of the alkenyl having 2 to 20 carbon atoms include the aforementioned linear alkenyl having 2 to 20 carbon atoms.

Examples of the substituent in the substituted alkyl or the substituted alkenyl represented by $R_a$ include a phenyl group, a hydroxy group, a sulfo group, a cyano group, and halogen atoms. Examples of the phenyl group-substituted alkyl include benzyl and 1-phenylethyl. Examples of the halogen atoms include chlorine, bromine, and iodine atoms.

When the pyridine ring has two or more substituents, these substituents may be the same or different. $X^-$ in compound (II) represents a monovalent anion. Examples of the monovalent anion include anions such as halogen ions, $OH^-$, $PF_6^-$, $BF_4^-$, $CH_3CH_2OSO_3^-$, and $(CF_3SO_2)_2N^-$. Examples of the halogen ions include $Cl^-$, $Br^-$, and $I^-$.

Specific examples (products) of compound (II) include 1-dodecylpyridinium chloride (hereinafter, referred to as C12py; manufactured by Tokyo Chemical Industry Co., Ltd.), 1-cetylpyridinium chloride, 1-cetyl-4-methylpyridinium chloride (manufactured by Tokyo Chemical Industry Co., Ltd.), and N-octadecyl-4-stilbazole bromide (manufactured by Tokyo Chemical Industry Co., Ltd.).

A phosphonium salt represented by the following general formula (III) [hereinafter, referred to as compound (III)] is used as the phosphonium salt;

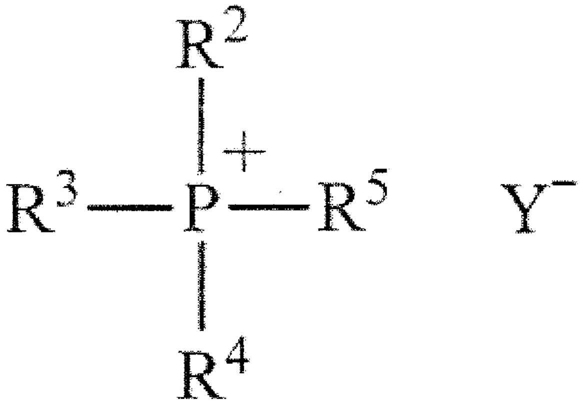

( III )

wherein $R^2$ to $R^5$ are the same or different and each represent substituted or unsubstituted alkyl; and $Y^-$ represents a monovalent anion.

Examples of alkyl in the substituted or unsubstituted alkyl represented by $R^2$ include linear alkyl having 8 to 20 carbon atoms, and branched alkyl having 8 to 20 carbon atoms. Examples of the linear alkyl having 8 to 20 carbon atoms include the aforementioned linear alkyl having 8 to 20 carbon atoms. Examples of the branched alkyl having 8 to 20 carbon atoms include the aforementioned branched alkyl having 8 to 20 carbon atoms. Examples of the substituent in the substituted alkyl include a phenyl group, a hydroxy group, a sulfo group, a cyano group, and halogen atoms. Examples of the phenyl group-substituted alkyl include benzyl and 1-phenylethyl. Examples of the halogen atoms include chlorine, bromine, and iodine atoms.

Examples of alkyl in the substituted or unsubstituted alkyl represented by each of $R^3$ to $R^5$ include linear alkyl having 1 to 20 carbon atoms, and branched alkyl having 3 to 20 carbon atoms. Examples of the linear alkyl having 1 to 20 carbon atoms include the aforementioned linear alkyl having 1 to 20 carbon atoms. Examples of the branched alkyl having 3 to 20 carbon atoms include the aforementioned branched alkyl having 3 to 20 carbon atoms. Examples of the substituent in the substituted alkyl include a phenyl group, a hydroxy group, a sulfo group, a cyano group, and halogen atoms. Examples of the phenyl group-substituted alkyl include benzyl and 1-phenylethyl. Examples of the halogen atoms include chlorine, bromine, and iodine atoms.

$Y^-$ represents a monovalent anion. Examples of the monovalent anion include anions such as halogen ions, $OH^-$, $PF_6^-$, $BF_4^-$, $CH_3CH_2OSO_3^-$, $(CF_3SO_2)_2N^-$, $B(C_6H_5)_4^-$, and benzotriazolate. Examples of the halogen ions include $Cl^-$, $Br^-$, and $I^-$.

Specific examples (products) of compound (III) include tetraoctylphosphonium bromide (manufactured by Tokyo Chemical Industry Co., Ltd.), tributyloctylphosphonium bromide (manufactured by Tokyo Chemical Industry Co., Ltd.), tributyldodecylphosphonium bromide, and tributylhexadecylphosphonium bromide.

An imidazolium salt represented by the following general formula (IV) [hereinafter, referred to as compound (IV)] is used as the imidazolium salt:

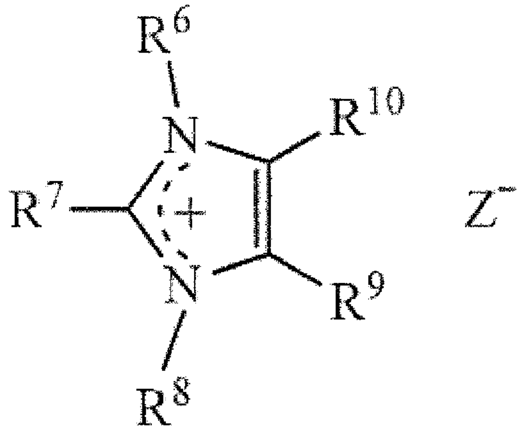

( IV )

wherein $R^6$ and $R^8$ are the same or different and each represent substituted or unsubstituted alkyl or substituted or unsubstituted alkenyl; $R^7$, $R^9$, and $R^{10}$ each represent a hydrogen atom, substituted or unsubstituted alkyl, or substituted or unsubstituted alkenyl; and $Z^-$ represents a monovalent anion.

Examples of alkyl in the substituted or unsubstituted alkyl represented by $R^6$ include linear alkyl having 8 to 20

carbon atoms, and branched alkyl having 8 to 20 carbon atoms. Examples of the linear alkyl having 8 to 20 carbon atoms include the aforementioned linear alkyl having 8 to 20 carbon atoms. Examples of the branched alkyl having 8 to 20 carbon atoms include the aforementioned branched alkyl having 8 to 20 carbon atoms.

Examples of alkenyl in the substituted or unsubstituted alkenyl represented by $R^6$ include alkenyl having 8 to 20 carbon atoms. Examples of the alkenyl having 8 to 20 carbon atoms include the aforementioned alkenyl having 8 to 20 carbon atoms.

Examples of the substituent in the substituted alkyl or the substituted alkenyl represented by $R^6$ include a phenyl group, a hydroxy group, a sulfo group, a cyano group, and halogen atoms. Examples of the phenyl group-substituted alkyl include benzyl and 1-phenylethyl. Examples of the halogen atoms include chlorine, bromine, and iodine atoms.

Examples of alkyl in the substituted or unsubstituted alkyl represented by each of $R^7$, $R^8$, $R^9$, and $R^{10}$ include linear alkyl having 1 to 20 carbon atoms, and branched alkyl having 3 to 20 carbon atoms. Examples of the linear alkyl having 1 to 20 carbon atoms include the aforementioned linear alkyl having 1 to 20 carbon atoms. Examples of the branched alkyl having 3 to 20 carbon atoms include the aforementioned branched alkyl having 3 to 20 carbon atoms.

Examples of alkenyl in the substituted or unsubstituted alkenyl represented by each of $R^7$, $R^8$, $R^9$, and $R^{10}$ include alkenyl having 2 to 20 carbon atoms. Examples of the alkenyl having 2 to 20 carbon atoms include the aforementioned alkenyl having 2 to 20 carbon atoms.

Examples of the substituent in the substituted alkyl or the substituted alkenyl represented by each of $R^7$, $R^8$, $R^9$, and $R^{10}$ include a phenyl group, a hydroxy group, a sulfo group, a cyano group, and halogen atoms. Examples of the phenyl group-substituted alkyl include benzyl and 1-phenylethyl. Examples of the halogen atoms include chlorine, bromine, and iodine atoms.

$Z^-$ represents a monovalent anion. Examples of the monovalent anion include anions such as halogen ions, $OH^-$, $PF_6^-$, $BF_4^-$, $CH_3CH_2OSO_3^-$, $(CF_3SO_2)_2N^-$, $(CH_3O)_2P(=O)O^-$, $B(C_6H_5)_4^-$, $FeCl_4^-$, $CF_3BF_3^-$, $CF_3SO_3^-$, $(NC)_2N^-$, $CH_3(OCH_2CH_2)_2OSO_3^-$, $CH_3CH_2OSO_3^-$, $HSO_4^-$, and p-$CH_3C_6H_4SO_3^-$. Examples of the halogen ions include $Cl^-$, $Br^-$, and $I^-$.

Specific examples (products) of compound (IV) include 1-methyl-3-octylimidazolium bromide (manufactured by Tokyo Chemical Industry Co., Ltd.), 1-methyl-3-octylimidazolium chloride (manufactured by Tokyo Chemical Industry Co., Ltd.), and 1-dodecyl-2-methyl-3-benzylimidazolium chloride.

An isoquinolinium salt represented by the following general formula (V) [hereinafter, referred to as compound (V)] is used as the isoquinolinium salt:

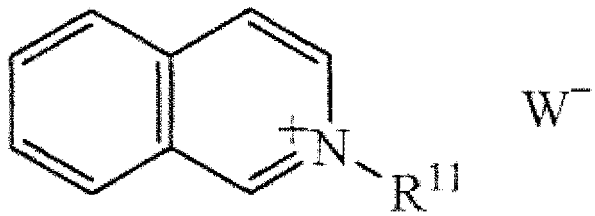

(V)

wherein $R^{11}$ represents substituted or unsubstituted alkyl or substituted or unsubstituted alkenyl; and $W^-$ represents a monovalent anion.

Examples of alkyl in the substituted or unsubstituted alkyl represented by $R^{11}$ include linear alkyl having 8 to 20 carbon atoms, and branched alkyl having 8 to 20 carbon atoms. Examples of the linear alkyl having 8 to 20 carbon atoms include the aforementioned linear alkyl having 8 to 20 carbon atoms. Examples of the branched alkyl having 8 to 20 carbon atoms include the aforementioned branched alkyl having 8 to 20 carbon atoms.

Examples of alkenyl in the substituted or unsubstituted alkenyl represented by $R^{11}$ include alkenyl having 8 to 20 carbon atoms. Examples of the alkenyl having 8 to 20 carbon atoms include the aforementioned alkenyl having 8 to 20 carbon atoms.

Examples of the substituent in the substituted alkyl or the substituted alkenyl represented by $R^{11}$ include a phenyl group, a hydroxy group, a sulfo group, a cyano group, and halogen atoms. Examples of the phenyl group-substituted alkyl include benzyl and 1-phenylethyl. Examples of the halogen atoms include chlorine, bromine, and iodine atoms.

$W^-$ represents a monovalent anion. Examples of the monovalent anion include anions such as halogen ions. Examples of the halogen ions include $Cl^-$, $Br^-$, and $I^-$.

Specific examples (products) of compound (V) include N-lauryl isoquinolinium chloride (manufactured by NOF Corp.) and N-lauryl isoquinolinium bromide (manufactured by NOF Corp.).

Examples of the anionic surfactants include sulfuric acid ester salt, carboxylate, sulfonate, phosphoric acid ester salt, sulfosuccinate, N-methyltaurine salt, and N-alkanoyl-N-methyltaurine salt.

Examples of the amphoteric surfactants include tertiary amine oxide and alkylcarboxybetaine.

Examples of the nonionic surfactants include polyoxyethylene alkylamine, polyoxyethylene alkenylamine, polyoxyethylene alkyl ether, polyoxyethylene alkenyl ether, polyoxyethylene alkylphenyl ether, ethylenediamine tetrapolyoxyethylene, and polyglycerin fatty acid ester. Polyoxyethylene alkylamine or polyoxyethylene alkyl ether is preferable.

Examples of alkyl in the polyoxyethylene alkylamine include alkyl having 8 to 20 carbon atoms. Examples of the alkyl having 8 to 20 carbon atoms include octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl.

Examples of alkenyl in the polyoxyethylene alkenylamine include alkenyl having 8 to 20 carbon atoms. Examples of the alkenyl having 8 to 20 carbon atoms include octenyl, nonenyl, decenyl, undecenyl, dodecenyl, tetradecenyl, pentadecenyl, hexadecenyl, heptadecenyl, octadecenyl, oleyl, nonadecenyl, and icosenyl.

Examples of alkyl in the polyoxyethylene alkyl ether include alkyl having 8 to 20 carbon atoms. Examples of the alkyl having 8 to 20 carbon atoms include the aforementioned alkyl having 8 to 20 carbon atoms.
Examples of alkenyl in the polyoxyethylene alkenyl ether include alkenyl having 8 to 20 carbon atoms. Examples of the alkenyl having 8 to 20 carbon atoms include the aforementioned alkenyl having 8 to 20 carbon atoms.

Examples of alkyl in the polyoxyethylene alkylphenyl ether include alkyl having 8 to 20 carbon atoms. Examples of the alkyl having 8 to 20 carbon atoms include the aforementioned alkyl having 8 to 20 carbon atoms.

The amount of total hemoglobin can be determined by a method known in the art, for example, cyanmethemoglobin method, oxyhemoglobin method, or SLS-hemoglobin method. The amount of total hemoglobin can be determined by applying the cyanmethemoglobin method, the oxyhemoglobin method, or the SLS-hemoglobin method not only to the hemoglobin-containing sample itself but to a hemoglobin-containing sample added an isothiazolinone derivative and/or a surfactant thereto, or a hemoglobin-containing sample added an isothiazolinone derivative and/or a surfactant and a proteolytic enzyme thereto.

The reaction of glycated hemoglobin in the hemoglobin-containing sample with a proteolytic enzyme in the presence of the surfactant can be performed under any condition as long as the proteolytic enzyme can react to glycated hemoglobin in the presence of the surfactant. The reaction of glycated hemoglobin in the hemoglobin-containing sample with a proteolytic enzyme is preferably performed in the aqueous medium. Examples of the aqueous medium include an aqueous medium described later. The reaction of glycated hemoglobin in the hemoglobin-containing sample with a proteolytic enzyme is performed usually at 10 to 50°C, preferably 20 to 40°C, and usually for 1 minute to 3 hours, preferably 2.5 minutes to 1 hour. The concentration of the proteolytic enzyme is not particularly limited as long as the reaction of glycated hemoglobin in the hemoglobin-containing sample with the proteolytic enzyme proceeds. The concentration is usually 50 to 25000 kU/L, preferably 250 to 10000 kU/L.

The proteolytic enzyme is not particularly limited as long as the enzyme reacts to glycated hemoglobin in the hemoglobin-containing sample to generate a glycated peptide from the glycated hemoglobin. Examples thereof include serine protease (chymotrypsin, subtilisin, etc.), cysteine protease (papain, caspase, etc.), aspartic acid protease (pepsin, cathepsin D, etc.), metalloprotease (thermolysin, etc.), N-terminal threonine protease, and glutamic acid protease. In the present invention, a commercially available proteolytic enzyme can be used. Examples of the commercially available product include Protease P "Amano" 3G and Protease K "Amano" (both manufactured by Amino Enzyme Inc.), Actinase AS and Actinase E (both manufactured by Kaken Pharma Co., Ltd.), Thermolysin (manufactured by Daiwa Fine Chemicals Co., Ltd.), and Sumizyme MP (manufactured by Shin Nihon Chemical Co., Ltd.).

The concentration of the surfactant in the reaction of a proteolytic enzyme is not particularly limited as long as the reaction of glycated hemoglobin in the hemoglobin-containing sample with the proteolytic enzyme proceeds. The concentration is usually 0.0001 to 10%, preferably 0.0005 to 5%.

The reaction of glycated hemoglobin in the hemoglobin-containing sample with the proteolytic enzyme generates a reaction product comprising a glycated peptide. Subsequently, the glycated peptide in the reaction product reacts with fructosyl peptide oxidase to generate hydrogen peroxide. The reaction of the glycated peptide with fructosyl peptide oxidase is preferably performed in an aqueous medium. Examples of the aqueous medium include an aqueous medium described later.

The reaction of the glycated peptide with fructosyl peptide oxidase is performed usually at 10 to 50°C, preferably 20 to 40°C, and usually for 1 minute to 3 hours, preferably 2.5 minutes to 1 hour. The concentration of the fructosyl peptide oxidase is not particularly limited as long as the reaction of the glycated hemoglobin with the fructosyl peptide oxidase proceeds. The concentration is usually 0.1 to 30 kU/L, preferably 0.2 to 15 kU/L.

The fructosyl peptide oxidase is not particularly limited as long as the enzyme acts on the glycated peptide to generate hydrogen peroxide. Examples thereof include fructosyl peptide oxidases derived from filamentous bacteria, yeasts, actinomycetes, bacteria, or archaebacteria. In the present invention, commercially available fructosyl peptide oxidase may be used. Examples of the commercially available product include FPOX-CE (manufactured by Kikkoman Corp.), FPOX-EE (manufactured by Kikkoman Corp.), and FPOX-CET (manufactured by Kikkoman Corp.).

The methods for measuring the generated hydrogen peroxide include a method using an electrode, and a method using a hydrogen peroxide measuring reagent. A method using a hydrogen peroxide measuring reagent is preferable. The hydrogen peroxide measuring reagent refers to a reagent for converting hydrogen peroxide to a detectable substance. Examples of the detectable substance include dyes, light (luminescence), and fluorescence. A dye is preferable.

In the case where the detectable substance is a dye, examples of the hydrogen peroxide measuring reagent include reagents comprising a peroxidatively active substance such as peroxidase and a chromogen capable of developing color by oxidation. Examples of the chromogen capable of developing color by oxidation include oxidative coupling-type chromogens and leuco chromogens. A leuco chromogen is preferable. Examples of the leuco chromogen include phenothiazine chromogens, triphenylmethane chromogens, diphenylamine chromogens, o-phenylenediamine, hydroxypropionic acid, diaminobenzidine, and tetramethylbenzidine. A phenothiazine chromogen is preferable. Examples of the phenothiazine chromogen include 10-N-carboxymethylcarbamoyl-3,7-bis(dimethylamino)-10H-phenothiazine (CCAP), 10-N-methylcarbamoyl-3,7-bis(dimethylamino)-10H-phenothiazine (MCDP), and 10-N-(carboxymethylaminocarbonyl)-3,7-bis(dimethylamino)-10H-phenothiazine sodium salt (DA-67). Among these phenothiazine chromogens, 10-N-(carboxymethylaminocarbonyl)-3,7-bis(dimethylamino)-10H-phenothiazine sodium salt (DA-67) is particularly preferable. Examples of the triphenylmethane chromogens include N,N,N',N',N",N"-hexa(3-sulfopropyl)-4,4',4"-triaminotriphenylmethane (TPM-PS). Examples of the diphenylamine chromogens include N-(carboxymethylaminocarbonyl)-4,4'-bis(dimethylaminc)diphenylamine sodium salt (DA-64), 4,4'-bis(dimethylamino)diphenylamine, and bis[3-bis(4-chlorophenyl)methyl-4-dimethylaminophenyl]amine (BCMA).

In the case where the detectable substance is light (luminescence), examples of the hydrogen peroxide measuring reagent include reagents comprising a peroxidatively active substance such as peroxidase and a chemiluminescent substance. Examples of the chemiluminescent substance include luminol, isoluminol, lucigenin, and acridinium ester.

In the case where the detectable substance is fluorescence, examples of the hydrogen peroxide measuring reagent include reagents comprising a peroxidatively active substance such as peroxidase and a fluorescent substance. Examples of the fluorescent substance include 4-hydroxyphenylacetic acid, 3-(4-hydroxyphenyl)propionic acid, and coumarin.

(2) Reagent for measuring glycated hemoglobin in hemoglobin-containing sample

The reagent for measuring glycated hemoglobin in a hemoglobin-containing sample according to the present invention comprises a proteolytic enzyme, fructosyl peptide oxidase, isothiazolinone derivative, and surfactant. The measuring reagent of the present invention is used in the method for measuring glycated hemoglobin in a hemoglobin-containing sample according to the present invention. The measurement reagent of the present invention can further comprise a hydrogen peroxide measuring reagent.

Examples of the proteolytic enzyme, the fructosyl peptide oxidase, isothiazolinone derivative, surfactant, and the hydrogen peroxide measuring reagent in the measuring reagent of the present invention include the aforementioned proteolytic enzyme, the fructosyl peptide oxidase, isothiazolinone derivative, surfactant, and the hydrogen peroxide measuring reagent, respectively.

A concentration of the proteolytic enzyme in the measuring reagent of the present invention is usually 50 to 25000 kU/L, preferably 250 to 10000 kU/L. A concentration of the fructosyl peptide oxidase in the measuring reagent of the present invention is usually 0.1 to 30 kU/L, preferably 0.2 to 15 kU/L.

A concentration of the isothiazolinone derivative in the measuring reagent of the present invention is usually 0.005 to 20 mmol/L, preferably 0.01 to 10 mmol/L.

A concentration of the surfactant in the measuring reagent of the present invention is usually 0.0001 to 10%, preferably 0.0005 to 5%.

The measuring reagent of the present invention can optionally comprise an aqueous medium, a stabilizer, an antiseptic, salts, an interference inhibitor, an organic solvent, and the like.

Examples of the aqueous medium include deionized water, distilled water, and buffer solution. A buffer solution is preferable.

The pH of the aqueous medium is, for example, 4 to 10. In the case of using a buffer solution as the aqueous medium, a buffer is preferably used according to the set pH. Examples of the buffer used in the buffer solution include a tris(hydroxymethyl)aminomethane buffer, a phosphate buffer, a borate buffer, and Good's buffers.

Examples of the Good's buffers include 2-morpholinoethanesuifonic acid (MES), bis(2-hydroxyethyl)iminotris (hydroxymethyl)methane (Bis-Tris), N-(2-acetamido)iminodiacetic acid (ADA), piperazine-N,N'-bis(2-ethanesulfonic acid) (PIPES), N-(2-acetamido)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), N-[tris(hydroxymethyl)methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid [(H)EPPS], N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO), and N-cyclohexyl-3-aminopropanesulfonic acid (CAPS).

A concentration of the buffer solution is usually 0.001 to 2.0 mol/L, preferably 0.005 to 1.0 mol/L.

Examples of the stabilizer include ethylenediaminetetraacetic acid (EDTA), sucrose, calcium chloride, calcium acetate, calcium nitrate, potassium ferrocyanide, bovine serum albumin (BSA), and polyoxyethylene surfactants such as polyoxyethylene alkylphenyl ether. Examples of the antiseptic include sodium azide and antibiotics. Examples of the salts include sodium chloride, sodium nitrate, sodium sulfate, sodium carbonate, sodium formate, sodium acetate, potassium chloride, potassium nitrate, potassium sulfate, potassium carbonate, potassium formate, and potassium acetate. Examples of the interference inhibitor include ascorbic acid oxidase for eliminating the influence of ascorbic acid. Examples of the organic solvent include solubilizers that make the leuco chromogen soluble in the aqueous medium, such as dimethylformamide (DMF), dimethyl sulfoxide (DMSO), dioxane, acetone, methanol, and ethanol.

(3) Kit for measuring glycated hemoglobin in hemoglobin-containing sample

The reagent for measuring glycated hemoglobin in a hemoglobin-containing sample according to the present invention can be stored, distributed, and used in the form of a kit. The kit for measuring glycated hemoglobin in a hemoglobin-containing sample according to the present invention is used in the method for measuring glycated hemoglobin in a hemoglobin-containing sample according to the present invention. Examples of the measuring kit of the present invention include a kit consisting of two reagents and a kit consisting of three reagents. A kit consisting of two reagents is preferable.

The kit for measuring glycated hemoglobin in a hemoglobin-containing sample according to the present invention is not particularly limited as long as the kit enables the method for measuring glycated hemoglobin in a hemoglobin-containing sample according to the present invention. Examples of kits consisting of two reagents include a kit comprising: a first reagent comprising a proteolytic enzyme; and a second reagent comprising fructosyl peptide oxidase, an isothiazolinone derivative, and a surfactant; and a kit comprising: a first reagent comprising a proteolytic enzyme, an isothiazolinone derivative, and a surfactant; and a second reagent comprising fructosyl peptide oxidase.

Further examples of the kit for measuring glycated hemoglobin according to the present invention include the kits described above, wherein either the first reagent or the second reagent, or both of the first reagent and the second reagent, comprise a hydrogen peroxide measuring reagent. Particularly, in the case of using a hydrogen peroxide measuring reagent comprising peroxidase and a leuco chromogen, the peroxidase and the leuco chromogen are preferably contained in separate reagents. Specifically, the peroxidase and the leuco chromogen are preferably contained in the first reagent and the second reagent or the second reagent and the first reagent, respectively.

A concentration of the proteolytic enzyme in the reagent constituting the measuring kit of the present invention is usually 100 to 30000 kU/L, preferably 500 to 10000 kU/L. A concentration of the fructosyl peptide oxidase in the reagent constituting the measuring kit of the present invention is usually 0.5 to 100 kU/L, preferably 1 to 50 kU/L. A concentration of the isothiazolinone derivative in the reagent constituting the measuring kit of the present invention is usually 0.005 to 20 mmol/L, preferably 0.01 to 10 mmol/L.

A concentration of the surfactant in the reagent constituting the measuring kit of the present invention is usually 0.0001 to 40%, preferably 0.0005 to 20%.

Hereinafter, the present invention will be described in more detail with reference to Examples. However, the scope of the present invention is not limited to these examples by any means.
In Examples, Comparative Examples, and Test Examples below, reagents and enzymes from the following manufacturers were used.

Bis-Tris (manufactured by Dojindo Laboratories), ADA (manufactured by Dojindo Laboratories), MES (manufactured by Dojindo Laboratories), calcium chloride dihydrate (manufactured by Wako Pure Chemical Industries, Ltd.), calcium acetate (manufactured by Wako Pure Chemical Industries, Ltd.), calcium nitrate (manufactured by Wako Pure Chemical Industries, Ltd.), DA-67 (manufactured by Wako Pure Chemical Industries, Ltd.), 1-dodecylpyridinium chloride (C12py) [compound (II); manufactured by Tokyo Chemical Industry Co., Ltd.], decyl trimethyl ammonium bromide (C10TMA) (quaternary ammonium salt; manufactured by Tokyo Chemical Industry Co., Ltd.), NIKKOL LMT (LMT) [N-alkanoyl-N-methyltaurine salt (N-lauroyl-N-methyltaurine sodium salt); manufactured by Nikko Chemicals Co., Ltd.], Anon BL [alkylcarboxybetaine (betaine lauryldimethylaminoacetate); manufactured by NOF Corp.], 2-octyl-4-isothiazolin-3-one (isothiazolinone derivative; manufactured by Tokyo Chemical Industry Co., Ltd.), 1,2-benzisothiazol-3(2H)-one (isothiazolinone derivative; manufactured by Wako Pure Chemical Industries, Ltd.), 4,5-dichloro-2-octyl-4-isothiazolin-3-one (manufactured by HighChem Co., Ltd.), Thermolysin (proteolytic enzyme; manufactured by Daiwa Fine Chemicals Co., Ltd.), Actinase E (proteolytic enzyme; manufactured by Kaken Pharma Co., Ltd.), FPOX-CE (fructosyl peptide oxidase; manufactured by Kikkoman Corp.), FPOX-CET (fructosyl peptide oxidase; manufactured by Kikkoman Corp.), peroxidase (manufactured by Toyobo Co., Ltd.), and Triton X-405 (polyoxyethylene alkylphenyl ether; manufactured by Sigma-Aldrich Corp.).

Example

A kit for HbA1c measurement consisting of the following first and second reagents was prepared.

| | |
|---|---|
| First reagent | |
| Bis-Tris (pH 6.8) | 10 mmol/L |
| Cl2py | 1.6 g/L |
| 2-octyl-4-isothiazolin-3-one | 0.2 g/L |
| Calcium chloride dihydrate | 10 mmol/L |
| Thermolysin | 1800 kU/L |
| Peroxidase | 40 kU/L |
| Second reagent | |
| ADA (pH 7.0) | 50 mmol/L |
| FPOX-CE | 6 kU/L |
| DA-67 | 60 μmol/L |

Example 2

A kit for HbA1c measurement consisting of the following first and second reagents was prepared.

| | |
|---|---|
| First reagent | |
| Bis-Tris (pH 6.8) | 10 mmol/L |
| C12py | 1.6 g/L |
| 1,2-benzisothiazol-3(2H)-one | 0.4 g/L |
| Calcium chloride dihydrate | 10 mmol/L |
| Thermolysin | 1800 kU/L |
| Peroxidase | 40 kU/L |
| Second reagent | |
| ADA (pH 7.0) | 50 mmol/L |
| FPOX-CE | 6 kU/L |
| DA-67 | 60 μmol/L |

Example 3

A kit for HbA1c measurement consisting of the following first and second reagents was prepared.

| | |
|---|---|
| First reagent | |
| Bis-Tris (pH 6.8) | 10 mmol/L |
| C10TMA | 16 g/L |
| 2-octyl-4-isothiazolin-3-one | 0.2 g/L |
| Calcium chloride dihydrate | 10 mmol/L |
| Thermolysin | 1800 kU/L |
| Peroxidase | 40 kU/L |
| Second reagent | |
| ADA (pH 7.0) | 50 mmol/L |
| FPOX-CE | 6 kU/L |
| ADA-67 | 60 μmol/L |

Example 4

A kit for HbA1c measurement consisting of the following first and second reagents was prepared.

First reagent

| | |
|---|---|
| Bis-Tris (pH 6.8) | 10 mmol/L |
| C10TMA | 16 g/L |
| 1,2-benzisothiazol-3(2H)-one | 0.2 g/L |
| Calcium chloride dihydrate | 10 mmol/L |
| Thermolysin | 1800 kU/L |
| Peroxidase | 40 kU/L |

Second reagent

| | |
|---|---|
| ADA (pH 7.0) | 50 mmol/L |
| FPOX-CE | 6 kU/L |
| ADA-67 | 60 μmol/L |

Example 5

A kit for HbA1c measurement consisting of the following first and second reagents was prepared.

First reagent

| | |
|---|---|
| Bis-Tris (pH 6.8) | 10 mmol/L |
| LMT | 5 g/L |
| 2-octyl-4-isothiazolin-3-one | 0.4 g/L |
| Calcium chloride dihydrate | 10 mmol/L |
| Thermolysin | 1800 kU/L |
| Peroxidase | 40 kU/L |

Second reagent

| | |
|---|---|
| ADA (pH 7.0) | 50 mmol/L |
| FPOX-CE | 6 kU/L |
| DA-67 | 60 μmol/L |

Example 6

A kit for HbA1c measurement consisting of the following first and second reagents was prepared.

First reagent

| | |
|---|---|
| Bis-Tris (pH 6.8) | 10 mmol/L |
| LMT | 5 g/L |
| 1,2-benzisothiazol-3(2H)-one | 0.2 g/L |
| Calcium chloride dihydrate | 10 mmol/L |
| Thermolysin | 1800 kU/L |
| Peroxidase | 40 kU/L |

Second reagent

| | |
|---|---|
| ADA (pH 7.0) | 50 mmol/L |
| FPOX-CE | 6 kU/L |
| DA-67 | 60 μmol/L |

Example 7

A kit for HbA1c measurement consisting of the following first and second reagents was prepared.

First reagent

| | |
|---|---|
| Bis-Tris (pH 6.8) | 10 mmol/L |
| Anon BL | 5 g/L |
| 2-octyl-4-isothiazolin-3-one | 0.2 g/L |
| Calcium chloride dihydrate | 10 mmol/L |

(continued)

| | |
|---|---|
| First reagent | |
| Thermolysin | 1800 kU/L |
| Peroxidase | 40 kU/L |
| Second reagent | |
| ADA (pH 7.0) | 50 mmol/L |
| FPOX-CE | 6 kU/L |
| DA-67 | 60 μmol/L |

Example 8

A kit for HbA1c measurement consisting of the following first and second reagents was prepared.

| | |
|---|---|
| First reagent | |
| Bis-Tris (pH 6.8) | 10 mmol/L |
| Anon EL | 5 g/L |
| 1,2-benzisothiazol-3(2H)-one | 0.4 g/L |
| Calcium chloride dihydrate | 10 mmol/L |
| Thermolysin | 1800 kU/L |
| Peroxidase | 40 kU/L |
| Second reagent | |
| ADA (pH 7.0) | 50 mmol/L |
| FPOX-CE | 6 kU/L |
| DA-67 | 60 μmol/L |

Example 9

A kit for HbA1c measurement consisting of the following first and second reagents was prepared.

| | |
|---|---|
| First reagent | |
| Bis-Tris (pH 6.8) | 10 mmol/L |
| C12py | 1.6 g/L |
| 2-octyl-4-isothiazolin-3-one | 0.4 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 kU/L |
| Peroxidase | 120 kU/L |

Example 10

A kit for HbA1c measurement consisting of the following first and second reagents was prepared.

| | |
|---|---|
| First reagent | |
| Bis-Tris (pH 6.8) | 10 mmol/L |
| C12py | 1.6 g/L |
| 1,2-benzisothiazol-3(2H)-one | 0.2 g/L |
| Calcium chloride dihydrate | 10 mmol/L |
| Thermolysin | 1800 kU/L |
| DA-67 | 20 μmol/L |

(continued)

| | |
|---|---|
| Second reagent | |
| ADA (pH 7.0) | 50 mmol/L |
| FPOX-CE | 6 kU/L |
| Peroxidase | 120 kU/L |

Example 11

A kit for HbA1c measurement consisting of the following first and second reagents was prepared.

| | |
|---|---|
| First reagent | |
| Bis-Tris (pH 6.8) | 10 mmol/L |
| C10TMA | 16 g/L |
| 2-octyl-4-isothiazolin-3-one | 0.4 g/L |
| Calcium chloride dihydrate | 10 mmol/L |
| Thermolysin | 1800 kU/L |
| DA-67 | 20 mmol/L |
| Second reagent | |
| ADA (pH 7.0) | 50 mmol/L |
| FPOX-CE | 6 kU/L |
| Peroxidase | 120 kU/L |

Example 12

A kit for HbA1c measurement consisting of the following first and second reagents was prepared.

| | |
|---|---|
| First reagent | |
| Bis-Tris (pH 6.8) | 10 mmol/L |
| C10TMA | 16 g/L |
| 1,2-benzisothiazol-3(2H)-one | 0.2 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 kU/L |
| Peroxidase | 120 kU/L |

Example 13

A kit for HbA1c measurement consisting of the following first and second reagents was prepared.

| | |
|---|---|
| First reagent | |
| Bis-Tris (pH 6.8) | 10 mmol/L |
| LMT | 5 g/L |
| 2-octyl-4-isothiazolin-3-one | 0.2 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |

(continued)

| | |
|---|---|
| Second reagent | |
| FPOX-CE | 6 kU/L |
| Peroxidase | 120 kU/L |

Example 14

A kit for HbA1c measurement consisting of the following first and second reagents was prepared.

| | |
|---|---|
| First reagent | |
| Bis-Tris (pH 6.8) | 10 mmol/L |
| LMT | 5 g/L |
| 1,2-benzisothiazol-3(2H)-one | 0.2 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 kU/L |
| Peroxidase | 120 kU/L |

Example 15

A kit for HbA1c measurement consisting of the following first and second reagents was prepared.

| | |
|---|---|
| First reagent | |
| Bis-Tris (pH 6.8) | 10 mmol/L |
| Anon BL | 5 g/L |
| 2-octyl-4-isothiazolin-3-one | 0.4 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 kU/L |
| Peroxidase | 120 kU/L |

Example 16

A kit for HbA1c measurement consisting of the following first and second reagents was prepared.

| | |
|---|---|
| First reagent | |
| Bis-Tris (pH 6.8) | 10 mmol/L |
| Anon BL | 5 g/L |
| 1,-benzisotriazol-3(2H)-one | 0.4 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 KU/L |

(continued)

| | |
|---|---|
| Second reagent | |
| Peroxidase | 120 kU/L |

Example 17

A kit for HbA1c measurement consisting of the following first and second reagents was prepared.

| | |
|---|---|
| First reagent | |
| MES (pH 6.0) | 20 mmol/L |
| C12py | 1.2 g/L |
| 2-octyl-4-isothiazolin-3-one | 0.2 g/L |
| Calcium acetate | 10 mmol/L |
| Sodium nitrate | 100 mmol/L |
| Actinase E | 340 kU/L |
| DA-67 | 20 μmol/L |
| Second reagent | |
| ADA (pH 7.0) | 50 mmol/L |
| FPOX-CET | 2.5 kU/L |
| Triton X-405 | 7.1 g/L |
| Peroxidase | 120 kU/L |

Example 18

A kit for HbA1c measurement consisting of the following first and second reagents was prepared.

| | |
|---|---|
| First reagent | |
| MES (pH 6.0) | 20 mmol/L |
| C12py | 1.2 g/L |
| 1,2-benzisothiazol-3(2H)-one | 0.04 g/L |
| Calcium acetate | 10 mmol/L |
| Sodium nitrate | 100 mmol/L |
| Actinase E | 340 kU/L |
| DA-67 | 20 μmol/L |
| Second reagent | |
| ADA (pH 7.0) | 50 mmol/L |
| FPOX-CET | 2.5 kU/L |
| Triton X-405 | 7.1 g/L |
| Peroxidase | 120 kU/L |

Example 19

A kit for HbA1c measurement consisting of the following first and second reagents was prepared.

| | |
|---|---|
| First reagent | |
| MES (pH 6.5) | 20 mmol/L |
| C12py | 1.6 g/L |
| 2-octyl-4-isothiazolin-3-one | 0.2 g/L |
| Calcium nitrate | 10 mmol/L |
| Sodium nitrate | 100 mmol/L |
| Thermolysin | 1800 kU/L |
| DA-67 | 20 μmol/L |

(continued)

| | |
|---|---|
| Second reagent | |
| ADA (pH 7.0) | 50 mmol/L |
| FPOX-CET | 6 kU/L |
| Triton X-405 | 7.1 g/L |
| Peroxidase | 120 kU/L |

Example 20

A kit for HbA1c measurement consisting of the following first and second reagents was prepared.

| | |
|---|---|
| First reagent | |
| MES (pH 6.5) | 20 mmol/L |
| C12py | 1.6 g/L |
| 4,5-dichloro-2-octyl-4-isothiazolin-3-one | 0.04 g/L |
| Calcium nitrate | 10 mmol/L |
| Sodium nitrate | 100 mmol/L |
| Thermolysin | 1800 kU/L |
| DA-67 | 20 μmol/L |
| Second reagent | |
| ADA (pH 7.0) | 50 mmol/L |
| FPOX-CET | 6 kU/L |
| Triton X-405 | 7.1 g/L |
| Peroxidase | 120 kU/L |

Example 21

A kit for HbA1c measurement consisting of the following first and second reagents was prepared.

| | |
|---|---|
| First reagent | |
| MES (pH 6.5) | 20 mmol/L |
| C12py | 1.6 g/L |
| 2-octyl-4-isothiazolin-3-one | 0.2 g/L |
| Calcium nitrate | 10 mmol/L |
| Sodium nitrate | 100 mmol/L |
| Actinase E | 340 kU/L |
| DA-67 | 20 μmol/L |
| Second reagent | |
| ADA (pH 7.0) | 50 mmol/L |
| FPOX-CET | 6 kU/L |
| Triton X-405 | 7.1 g/L |
| Peroxidase | 120 kU/L |

Example 22

A kit for HbA1c measurement consisting of the following first and second reagents was prepared.

| | |
|---|---|
| First reagent | |
| MES (pH 6.5) | 20 mmol/L |
| C12py | 1.6 g/L |
| 4,5-dichloro-2-octyl-4-isothiazolin-3-one | 0.04 g/L |
| Calcium nitrate | 10 mmol/L |

(continued)

| | |
|---|---|
| First reagent | |
| Sodium nitrate | 100 mmol/L |
| Actinase E | 340 kU/L |
| DA-67 | 20 μmol/L |
| Second reagent | |
| ADA (pH 7.0) | 50 mmol/L |
| FPOX-CET | 6 kU/L |
| Triton X-405 | 7.1 g/L |
| Peroxidase | 120 kU/L |

Example 23

The kit of Example 1 was used as a kit for HbA1c measurement. Whole blood derived from 10 test subjects suspected of having diabetes mellitus was used as a sample to determine the ratio [HbA1c (%)] of HbA1c concentration (amount) to total hemoglobin concentration (amount) in each sample by the following procedures:

(1) Preparation of calibration curve for determining total hemoglobin concentration

"Hemoglobin B-Test Wako" (SLS-hemoglobin method) (manufactured by Wako Pure Chemical Industries, Ltd.), was used as a kit for total hemoglobin measurement. A standard (hemoglobin concentration: 15.3 mg/mL) included in "Hemoglobin B-Test Wako" was used as a specimen in measurement to prepare a calibration curve showing the relationship between hemoglobin concentration and absorbance.

(2) Preparation of calibration curve for determining HbA1c concentration

For each of two blood cell fractions with HbA1c concentrations valued at 2.77 μmol/L and 6.33 μmol/L, respectively, by latex immunoagglutination assay and the total hemoglobin value for each blood cell fraction, the measurement was performed using the kit for HbA1c measuring of Example 1 to determine the absorbance for each blood cell fraction. Saline was used instead of the blood cell fraction to determine the HbA1c concentration for the saline. The absorbance for the saline was subtracted from the absorbance for each blood cell fraction, and the value thus calculated was used as the blank-corrected absorbance for the blood cell fraction. A calibration curve showing the relationship between HbA1c concentration (μmol/L) and absorbance was prepared from the blank-corrected absorbance for the blood cell fraction and the blank-corrected absorbance (0 Abs) for the saline.

(3) Determination of hemoglobin concentration in each blood cell fraction

Each sample was centrifuged at 3000 rpm at 25°C for 5 minutes to obtain a blood cell fraction. For each blood cell fraction, the measurement was performed using "Hemoglobin B-Test Wako", and the hemoglobin concentration (μmol/L) in each blood cell fraction was determined from the obtained measurment value and the calibration curve prepared in the paragraph (1).

(4) Determination of HbA1c concentration in each blood cell fraction

For each blood cell fraction, the measurement was performed using the measuring kit of Example 1. The HbA1c concentration (μmol/L) in each blood cell fraction was determined from the obtained measurment value and the calibration curve prepared in the paragraph (2).

(5) Determination of HbA1c (%) (= ratio of HbA1c concentration to hemoglobin concentration)

HbA1c (%) was calculated as a Japan Diabetes Society (JDS) value according to the following formula from the hemoglobin concentration (μmol/L) in each blood cell fraction determined in the paragraph (3) and the HbA1c concentration (μmol/L) in each blood cell fraction determined in the paragraph (4):

[Equation 1]

$$HbA1c(\%) = [HbA1c\text{ concentration }(\mu mol/L)]/[\text{Hemoglobin concentration }(\mu mol/L)] \times 0.0963 + 1.62$$

(6) Determination of HbA1c (%) in same blood cell fraction by immunoassay

The same blood cell fractions as those used in the determination of HbA1c (%) in the paragraph (5) were used. HbA1c (%) in each blood cell fraction was determined by immunoassay using "Determiner L HbA1c" (manufactured by Kyowa Medex Co., Ltd.) according to the protocol described in the attachment of "Determiner L HbA1c".

(7) Correlation between measuring method of the present invention and immunoassay

The correlation between the measuring method of the present invention and immunoassay was verified from HbA1c (%) determined in the paragraph (5) using the measuring method of the present invention and HbA1c (%) determined in the paragraph (6) using the immunoassay to determine a correlation coefficient.

The correlation coefficient between the measuring method of the present invention and a measurement using "Determiner L HbA1c" (manufactured by Kyowa Medex Co., Ltd.) was determined in the same way as above except that the measuring kits of Examples 2 to 22 were separately used instead of the measuring kit of Example 1. The results are shown in Table 1.

Table 1

| Kit | Correlation coefficient |
|---|---|
| Example 1 | 0.9967 |
| Example 2 | 0.9973 |
| Example 3 | 0.9949 |
| Example 4 | 0.9977 |
| Example 5 | 0.9978 |
| Example 6 | 0.9986 |
| Example 7 | 0.9935 |
| Example 8 | 0.9984 |
| Example 9 | 0.9979 |
| Example 10 | 0.9984 |
| Example 11 | 0.9976 |
| Example 12 | 0.9984 |
| Example 13 | 0.9965 |
| Example 14 | 0.9981 |
| Example 15 | 0.9936 |
| Example 16 | 0.9975 |
| Example 17 | 0.9980 |
| Example 18 | 0.9984 |
| Example 19 | 0.9969 |
| Example 20 | 0.9983 |
| Example 21 | 0.9967 |

(continued)

| Kit | Correlation coefficient |
|---|---|
| Example 22 | 0.9959 |

As shown in Table 1, favorable correlation was confirmed between the measuring method of the present invention using each of the measuring kits of Examples 1 to 22 and the immunoassay. These results demonstrated that the measuring method of the present invention using each of the measuring kits of Examples 1 to 22 could accurately and highly sensitively measure HbA1c in a sample.

[Comparative Example 1]

A kit for HbA1c measurement consisting of the following first and second reagents was prepared.

| | | |
|---|---|---|
| First reagent | | |
| | Bis-Tris (pH 6.8) | 10 mmol/L |
| | C12py | 1.6 g/L |
| | Calcium chloride dihydrate | 10 mmol/L |
| | Thermolysin | 1800 kU/L |
| | Peroxidase | 40 kU/L |
| Second reagent | | |
| | ADA (pH 7.0) | 50 mmol/L |
| | FPOX-CE | 6 kU/L |
| | DA-67 | 60 mmol/L |

[Comparative Example 2]

A kit for HbA1c measurement consisting of the following first and second reagents was prepared.

| | | |
|---|---|---|
| First reagent | | |
| | Bis-Tris (pH 6.8) | 10 mmol/L |
| | C10TMA | 16 g/L |
| | Calcium chloride dihydrate | 10 mmol/L |
| | Thermolysin | 1800 kU/L |
| | Peroxidase | 40 kU/L |
| Second reagent | | |
| | ADA (pH 7.0) | 50 mmol/L |
| | FPOX-CE | 6 kU/L |
| | DA-67 | 60 μmol/L |

[Comparative Example 3]

A kit for HbA1c measurement consisting of the following first and second reagents was prepared.

| | | |
|---|---|---|
| First reagent | | |
| | Bis-Tris (pH 6.8) | 10 mmol/L |
| | LMT | 5 g/L |
| | Calcium chloride dihydrate | 10 mmol/L |
| | Thermolysin | 1800 kU/L |
| | Peroxidase | 40 kU/L |
| Second reagent | | |
| | ADA (pH 7.0) | 50 mmol/L |

(continued)

| | | |
|---|---|---|
| Second reagent | | |
| | FPOX-CE | 6 kU/L |
| | DA-67 | 60 μmol/L |

[Comparative Example 4]

A kit for HbA1c measurement consisting of the following first and second reagents was prepared.

| | | |
|---|---|---|
| First reagent | | |
| | Bis-Tris (pH 6.8) | 10 mmol/L |
| | Anon BL | 5 g/L |
| | Calcium chloride dihydrate | 10 mmol/L |
| | Thermolysin | 1800 kU/L |
| | Peroxidase | 40 kU/L |
| Second reagent | | |
| | ADA (pH 7.0) | 50 mmol/L |
| | FPOX-CE | 6 kU/L |
| | ADA-67 | 60 mmol/L |

[Comparative Example 5]

A kit for HbA1c measurement consisting of the following first and second reagents was prepared.

| | | |
|---|---|---|
| First reagent | | |
| | Bis-Tris (pH 6.8) | 10 mmol/L |
| | C12py | 1.6 g/L |
| | Calcium chloride dihydrate | 10 mmol/L |
| | Thermolysin | 1800 kU/L |
| | DA-67 | 20 μmol/L |
| Second reagent | | |
| | ADA (pH 7.0) | 50 mmol/L |
| | FPOX-CE | 6 kU/L |
| | Peroxidase | 120 kU/L |

[Comparative Example 6]

A kit for HbA1c measurement consisting of the following first and second reagents was prepared.

| | | |
|---|---|---|
| First reagent | | |
| | Bis-Tris (pH 6.8) | 1.0 mmol/L |
| | C10TMA | 16 g/L |
| | Calcium chloride dihydrate | 10 mmol/L |
| | Thermolysin | 1800 kU/L |
| | DA-67 | 20 μmol/L |
| Second reagent | | |
| | ADA (pH 7.0) | 50 mmol/L |
| | FPOX-CE | 6 kU/L |
| | Peroxidase | 120 kU/L |

[Comparative Example 7]

A kit for HbA1c measurement consisting of the following first and second reagents was prepared.

| | |
|---|---|
| First reagent | |
| Bis-Tris (pH 6.8) | 10 mmol/L |
| LMT | 5 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 kU/L |
| Peroxidase | 120 kU/L |

[Comparative Example 8]

A kit for HbA1c measurement consisting of the following first and second reagents was prepared.

| | |
|---|---|
| First reagent | |
| Bis-Tris (pH 6.8) | 10 mmol/L |
| Anon BL | 5 g/L |
| Calcium chloride dihydrate | 10 mmol/L |
| Thermolysin | 1800 kU/L |
| DA-67 | 20 μmol/L |
| Second reagent | |
| ADA (pH 7.0) | 50 mmol/L |
| FPOX-CE | 6 kU/L |
| Peroxidase | 120 kU/L |

[Comparative Example 9]

A kit for HbA1c measurement consisting of the following first and second reagents was prepared.

| | |
|---|---|
| First reagent | |
| MES (pH 6.0) | 20 mmol/L |
| C12py | 1.2 g/L |
| Calcium acetate | 10 mmol/L |
| Sodium nitrate | 100 mmol/L |
| Actinase E | 340 kU/L |
| DA-67 | 20 μmol/L |
| Second reagent | |
| ADA (pH 7.0) | 50 mmol/L |
| FPOX-CET | 2.5 kU/L |
| Triton X-405 | 7.1 g/L |
| Peroxidase | 120 kU/L |

[Comparative Example 10]

A kit for HbA1c measurement consisting of the following first and second reagents was prepared.

| | |
|---|---|
| First reagent | |
| MES (pH 6.0) | 20 mmol/L |

(continued)

| | | |
|---|---|---|
| First reagent | | |
| | C12py | 1.6 g/L |
| | Calcium nitrate | 10 mmol/L |
| | Sodium nitrate | 100 mmol/L |
| | Thermolysin | 1800 kU/L |
| | DA-67 | 20 μmol/L |
| Second reagent | | |
| | ADA (pH 7.0) | 50 mmol/L |
| | FPOX-CET | 6 kU/L |
| | Triton X-405 | 7.1 g/L |
| | Peroxidase | 120 kU/L |

[Comparative Example 11]

A kit for HbA1c measurement consisting of the following first and second reagents was prepared.

| | | |
|---|---|---|
| First reagent | | |
| | MES (pH 6.5) | 20 mmol/L |
| | C12py | 1.6 g/L |
| | Calcium nitrate | 10 mmol/L |
| | Sodium nitrate | 100 mmol/L |
| | Actinase E | 340 kU/L |
| | ADA-67 | 20 μmol/L |
| Second reagent | | |
| | ADA (pH 7.0) | 50 mmol/L |
| | FPOX-CET | 6 kU/L |
| | Triton X-405 | 7.1 g/L |
| | Peroxidase | 120 kU/L |

[Test Example 1] Effect of isothiazolinone derivative (1)

(1) Preparation of hemolyzed specimen

For a blood cell fraction obtained by the centrifugation of human blood, the measurement was performed using a hemoglobin measuring reagent "Nescoat Hemo Kit-N" (manufactured by Alfresa Pharma Corp.) to determine the concentration of hemoglobin in the blood cell fraction. Subsequently, the blood cell fraction thus valued was hemolyzed by dilution with purified water to prepare each hemolyzed specimen having a hemoglobin concentration of 2 mg/mL, 4 mg/mL, 6 mg/mL, 8 mg/mL, or 10 mg/mL.

(2) Determination of reaction absorbance for hemolyzed specimen

The kit of Example 1 was used as a kit. Saline (hemoglobin concentration: 0 mg/mL) and the hemolyzed specimens prepared in the paragraph (1) were used as a specimen. The reaction absorbance for each specimen was determined by the following method:

9.6 μL of each specimen and 120 μL of the first reagent in the kit of Example 1 were added to a reaction cuvette and the mixture was incubated at 37°C for 5 minutes (first reaction). The absorbance (E1) of the reaction solution was determined at a primary wavelength of 660 nm and a secondary wavelength of 800 nm. Subsequently, 40 μL of the second reagent was added to this reaction solution, and the mixture was further incubated at 37°C for 5 minutes (second reaction). The absorbance (E2) of the reaction solution was determined at a primary wavelength of 660 nm and a secondary wavelength of 800 nm. E1 was subtracted from E2 to calculate an absorbance difference ΔE (=E2 - E1) as the reaction absorbance for each specimen.

The reaction absorbance for each specimen was determined in the same way as above except that the kits of Example 2 and Comparative Example 1 were separately used instead of the kit of Example 1. The results are shown in

Figure 1.

As is evident from Figure 1, the reaction absorbance increased in proportion to hemoglobin concentration in the measuring method of the present invention using the kit of Example 1 or 2 containing the isothiazolinone derivative, whereas no proportional relationship was observed between hemoglobin concentration and reaction absorbance in the measuring method using the kit of Comparative Example 1 containing no isothiazolinone derivative. Since the hemolyzed specimens used in the measurement were prepared from the same human blood, the HbA1c concentration increases depending on the hemoglobin concentration. Thus, the measuring method using the kit of Comparative Example 1 containing no isothiazolinone derivative hardly achieved accurate measurement of HbA1c because of being strongly influenced by hemoglobin. By contrast, the measuring method of the present invention using the kit of Example 1 or 2 containing the isothiazolinone derivative was shown to be capable of accurately measuring HbA1c without being influenced by hemoglobin.

Furthermore, the reaction absorbance for each specimen was determined in the same way as above except that the kits of Examples 9 and 10, and Comparative Example 5 were separately used instead of the kit of Example 1. The results are shown in Figure 2.

As is evident from Figure 2, the reaction absorbance increased in proportion to hemoglobin concentration in the measuring method of the present invention using the kit of Example 9 or 10 containing the isothiazolinone derivative, whereas no proportional relationship was observed between hemoglobin concentration and reaction absorbance in the measuring method using the kit of Comparative Example 5 containing no isothiazolinone derivative. Since the hemolyzed specimens used in the measurement were prepared from the same human blood, the HbA1c concentration increases depending on the hemoglobin concentration. Thus, the measuring method using the kit of Comparative Example 5 containing no isothiazolinone derivative hardly achieved accurate measurement of HbA1c because of being strongly influenced by hemoglobin. By contrast, the measuring method of the present invention using the kit of Example 9 or 10 containing the isothiazolinone derivative was shown to be capable of accurately measuring HbA1c without being influenced by hemoglobin.

[Test Example 2] Effect of isothiazolinone derivative (2)

The kit of Example 1 was used as a kit 1, and each of the hemolyzed specimens prepared in Test Example 1(1) was used as a specimen. On the basis of the obtained measurement value, the HbA1c concentration (μmol/L) in each specimen was determined from the calibration curve prepared in Example 9(2) representing the relationship between HbA1c concentration (μmol/L) and absorbance. Meanwhile, the hemoglobin concentration in each specimen was measured using "Hemoglobin B-Test Wako". The hemoglobin concentration (μmol/L) in each specimen was determined from the obtained measurement value and the calibration curve prepared in Example 9(1).

HbA1c (%) was calculated as a Japan Diabetes Society (JDS) value according to the following formula from the determined HbA1c concentration (μmol/L) and hemoglobin concentration (μmol/L) of each specimen:

[Equation 2]

$$HbA1c(\%) = [HbA1c\text{ concentration }(\mu\text{mol/L})]/[\text{Hemoglobin concentration }(\mu\text{mol/L})] \times 0.0963 + 1.62$$

The HbA1c concentration (%) in each specimen was determined by the same measurement using each kit except that the kits of Examples 2 to 13, 15, and 17 to 22 and Comparative Examples 1 to 11 were separately used instead of the kit of Example 1. The HbA1c concentration (%) of the specimen with a hemoglobin concentration of 6 mg/mL was used as a reference 0. A difference [ΔHbA1c concentration (%)] of the HbA1c concentration (%) from the reference was calculated for each specimen. The results are shown in Table 2.

Table 2

| Kit | ΔHbA1c concentration (%) | | | | |
|---|---|---|---|---|---|
| | Specimen [hemoglobin concentration (mg/mL)] | | | | |
| | 2 | 4 | 6 | 8 | 10 |
| Example 1 | 1.7 | 0.6 | 0.0 | -0.3 | -0.7 |
| Example 2 | -0.1 | 0.0 | 0.0 | 0.0 | 0.0 |
| Comparative Example 1 | 5.9 | 1.5 | 0.0 | -0.5 | -0.9 |
| Example 3 | 0.0 | 0.0 | 0.0 | 0.3 | 0.2 |
| Example 4 | 0.1 | -0.1 | 0.0 | 0.0 | 0.0 |
| Comparative Example 2 | 1.8 | 0.6 | 0.0 | -0.4 | -0.6 |
| Example 5 | -0.6 | -0.1 | 0.0 | 0.1 | 0.0 |
| Example 6 | -0.4 | -0.1 | 0.0 | 0.1 | 0.2 |
| Comparative Example 3 | -0.6 | 0.3 | 0.0 | -0.2 | -0.3 |
| Example 7 | -0.1 | -0.2 | 0.0 | 0.2 | 0.4 |
| Example 8 | 0.3 | 0.1 | 0.0 | -0.1 | -0.2 |
| Comparative Example 4 | 1.6 | 0.4 | 0.0 | -0.3 | -0.5 |
| Example 9 | 1.3 | 0.3 | 0.0 | -0.3 | -0.5 |
| Example 10 | 0.3 | 0.0 | 0.0 | -0.1 | -0.1 |
| Comparative Example 5 | 4.9 | 1.3 | 0.0 | -0.7 | -1.0 |
| Example 11 | 0.1 | -0.1 | 0.0 | 0.1 | 0.2 |
| Example 12 | 1.1 | 0.4 | 0.0 | -0.1 | -0.2 |
| Comparative Example 6 | 1.5 | 0.4 | 0.0 | -0.2 | -0.4 |
| Example 13 | -0.1 | 0.1 | 0.0 | -0.1 | -0.2 |
| Comparative Example 7 | -0.3 | -0.1 | 0.0 | -0.1 | -0.1 |
| Example 15 | 0.3 | -0.1 | 0.0 | 0.1 | 0.2 |
| Comparative Example 8 | 1.8 | 0.5 | 0.0 | -0.2 | -0.4 |
| Example 17 | 0.3 | 0.1 | 0.0 | -0.1 | -0.3 |
| Example 18 | 0.1 | -0.1 | 0.0 | -0.1 | -0.3 |
| Comparative Example 9 | 1.1 | 0.4 | 0.0 | -0.1 | -0.3 |
| Example 19 | 0.7 | 0.1 | 0.0 | -0.1 | -0.2 |
| Example 20 | 0.1 | 0.1 | 0.0 | -0.1 | -0.2 |
| Comparative Example 10 | 1.1 | 0.2 | 0.0 | -0.2 | -0.3 |
| Example 21 | 0.4 | 0.0 | 0.0 | 0.0 | -0.2 |
| Example 22 | -0.3 | -0.1 | 0.0 | -0.1 | -0.2 |
| Comparative Example 11 | 1.8 | 0.5 | 0.0 | -0.3 | -0.6 |

Since the hemolyzed specimens used in the measurement were prepared from the same human blood, as described above, the ratio (%) of HbA1c to total hemoglobin is constant, irrespective of hemoglobin concentration. Thus, ΔHbA1c concentration (%) closer to 0 represents that the HbA1c measuring method is less influenced by hemoglobin concentration. As is evident from Table 2, the kit of the present invention containing the isothiazolinone derivative was shown to be insusceptible to hemoglobin concentration compared with the kit of Comparative Example containing no isothiazolinone derivative.

[Test Example 3] Effect of isothiazolinone derivative (3)

Each of the kits of Examples 7 and 8 and Comparative Example 4 was used as a kit. Each of the hemolyzed specimens prepared in Test Example 1(1) was used as a specimen. The reaction absorbance for each specimen was determined in the same way as in Test Example 1 using each kit. The results are shown in Figure 3.

Likewise, each of the kits of Examples 15 and 16 and Comparative Example 8 was used as a kit, and each of the hemolyzed specimens prepared in Test Example 1(1) was used as a specimen. The reaction absorbance for each specimen was determined in the same way as in Test Example 1 using each kit. The results are shown in Figure 4.

Likewise, each of the kits of Examples 21 and 22 and Comparative Example 11 was used as a kit, and each of the hemolyzed specimens prepared in Test Example 1(1) was used as a specimen. The reaction absorbance for each specimen was determined in the same way as in Test Example 1 using each kit. The results are shown in Figure 5.

As is evident from Figures 3 and 4, the reaction absorbance increased in proportion to hemoglobin concentration in both the kit of the present invention and the kit of Comparative Example. However, the reaction absorbance was shown to be higher in the specimens having the same concentration of hemoglobin (i.e., the specimen having the same concentration of HbA1c) using the kit of the present invention containing the isothiazolinone derivative than using the kit of Comparative Example containing no isothiazolinone.

Since the hemolyzed specimens used in the measurement were prepared from the same human blood, as described above, the HbA1c concentration increases depending on the hemoglobin concentration. In the specimens having the same concentration of HbA1c, the higher reaction absorbance represents that the measurement data is less influenced by hemoglobin and is thus more reliable. Particularly, for a specimen having a low concentration of HbA1c, it is important to obtain high reaction absorbance. The high reaction absorbance was obtained in the specimens having the same concentration of HbA1c using the kits of Examples 7 and 8 compared with the kit of Comparative Example 4. Likewise, the high reaction absorbance was obtained in the specimens having the same concentration of HbA1c using the kits of Examples 15 and 16 compared with the kit of Comparative Example 8 as well as using the kits of Examples 21 and 22 compared with the kit of Comparative Example 11. These results demonstrated that the measuring method using the kit of the present invention containing the isothiazolinone derivative was capable of highly sensitively measuring HbA1c without being influenced by hemoglobin, compared with the measuring method using the kit of Comparative Example containing no isothiazolinone derivative.

## Industrial Applicability

The present invention provides a method, a reagent, and a kit for measuring glycated hemoglobin in a hemoglobin-containing sample, which are useful in, for example, measurement of glycated hemoglobin useful in the diagnosis of diabetes mellitus.

## Claims

1. A method for measuring glycated hemoglobin in a hemoglobin-containing sample, comprising reacting the hemoglobin-containing sample with a proteolytic enzyme in the presence of a surfactant, and then reacting the obtained reaction product with fructosyl peptide oxidase, wherein at least one of the former reaction and the latter reaction is performed in the presence of an isothiazolinone derivative; and measuring the generated hydrogen peroxide.

2. The method according to claim 1, wherein the isothiazolinone derivative is a compound represented by the following formula (I):

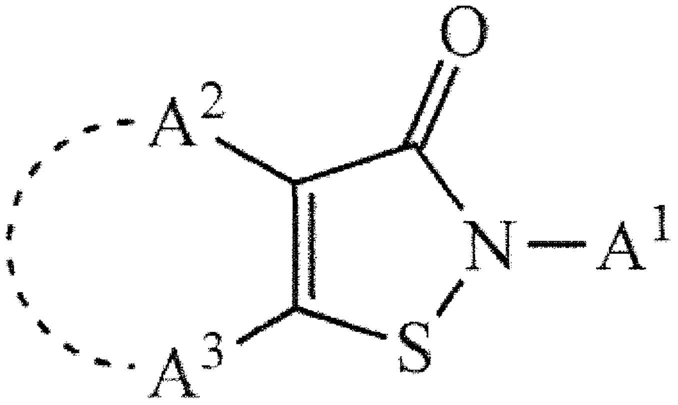

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

3. The method according to claim 2, wherein the compound represented by the formula (I) is an isothiazolinone derivative selected from the group consisting of 2-alkyl-4-isothiazolin-3-one, 1,2-benzisothiazol-3(2H)-one, and 2-alkyl-4,5-dihalogeno-4-isothiazolin-3-one.

4. The method according to any one of claims 1 to 3, wherein the measurement of the hydrogen peroxide is performed using a hydrogen peroxide measuring reagent.

5. The method according to claim 4, wherein the hydrogen peroxide measuring reagent is a reagent comprising peroxidase and a leuco chromogen.

6. A reagent for measuring glycated hemoglobin in a hemoglobin-containing sample, comprising a proteolytic enzyme, fructosyl peptide oxidase, an isothiazolinone derivative, and a surfactant.

7. The reagent according to claim 6, wherein the isothiazolinone derivative is a compound represented by the following formula (I):

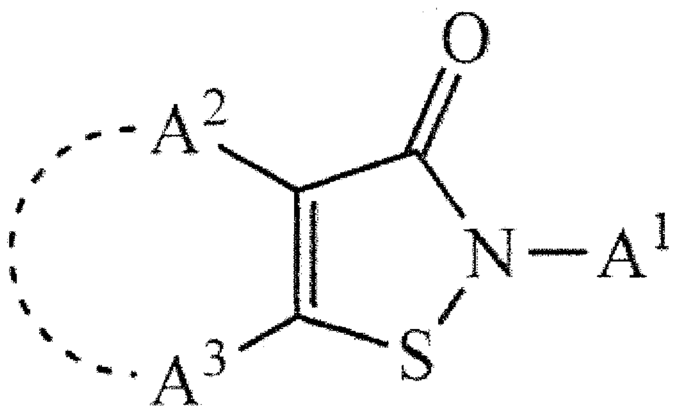

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

8. The reagent according to claim 7, wherein the compound represented by the formula (I) is an isothiazolinone derivative selected from the group consisting of 2-alkyl-4-isothiazolin-3-one, 1,2-benzisothiazol-3(2H)-one, and 2-alkyl-4,5-dihalogeno-4-isothiazolin-3-one.

**9.** The reagent according to any one of claims 6 to 8, further comprising a hydrogen peroxide measuring reagent.

**10.** The reagent according to claim 9, wherein the hydrogen peroxide measuring reagent is a reagent comprising peroxidase and a leuco chromogen.

**11.** A kit for measuring glycated hemoglobin in a hemoglobin-containing sample comprising: a first reagent comprising a proteolytic enzyme, an isothiazolinone derivative, and a surfactant; and a second reagent comprising fructosyl peptide oxidase.

**12.** A kit for measuring glycated hemoglobin in a hemoglobin-containing sample comprising: a first reagent comprising a proteolytic enzyme and a surfactant; and a second reagent comprising fructosyl peptide oxidase and an isothiazolinone derivative.

**13.** The kit according to claim 11 or 12, wherein the isothiazolinone derivative is a compound represented by the following formula (I):

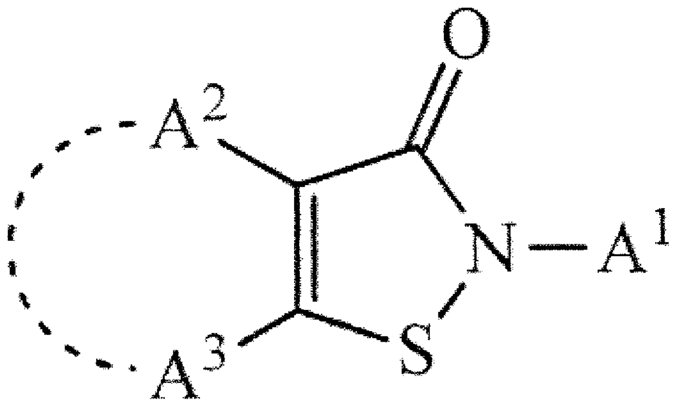

(I)

wherein $A^1$ represents a hydrogen atom or a substituted or unsubstituted alkyl; and $A^2$ and $A^3$ are the same or different, and each represents a hydrogen atom, a substituted or unsubstituted alkyl, or a halogen atom, or $A^2$ and $A^3$ together form a ring structure.

**14.** The kit according to claim 13, wherein the compound represented by the formula (I) is an isothiazolinone derivative selected from the group consisting of 2-alkyl-4-isothiazolin-3-one, 1,2-benzisothiazol-3(2H)-one, and 2-alkyl-4,5-dihalogeno-4-isothiazolin-3-one.

**15.** The kit according to any one of claims 11 to 14, wherein the first reagent and the second reagent or the second reagent and the first reagent further comprise peroxidase and a leuco chromogen, respectively.

[Figure 1]

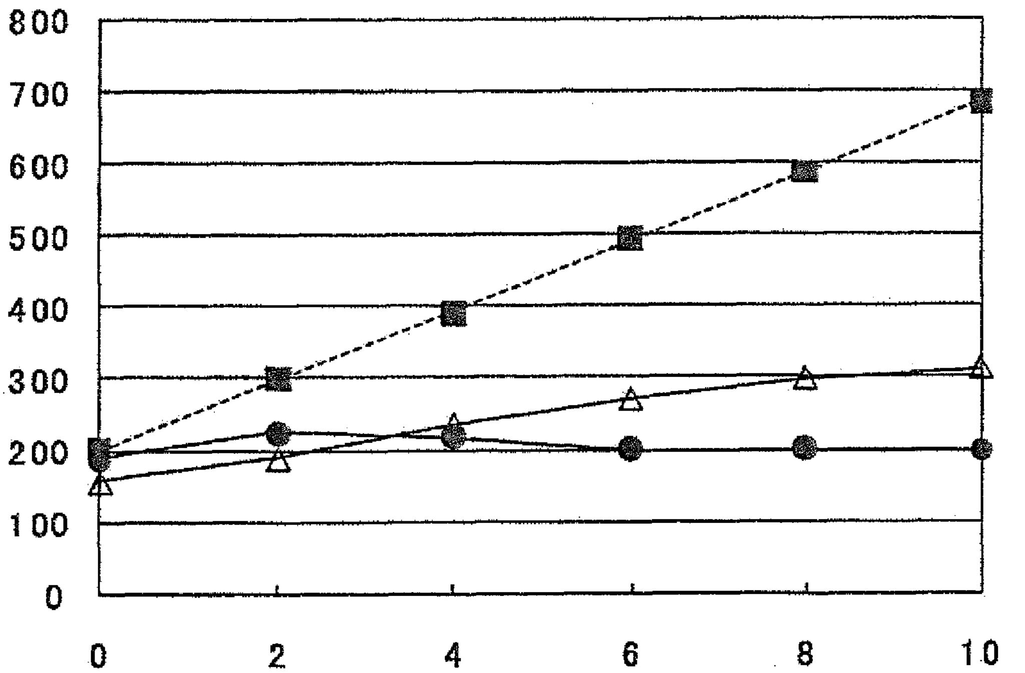


[Figure 2]

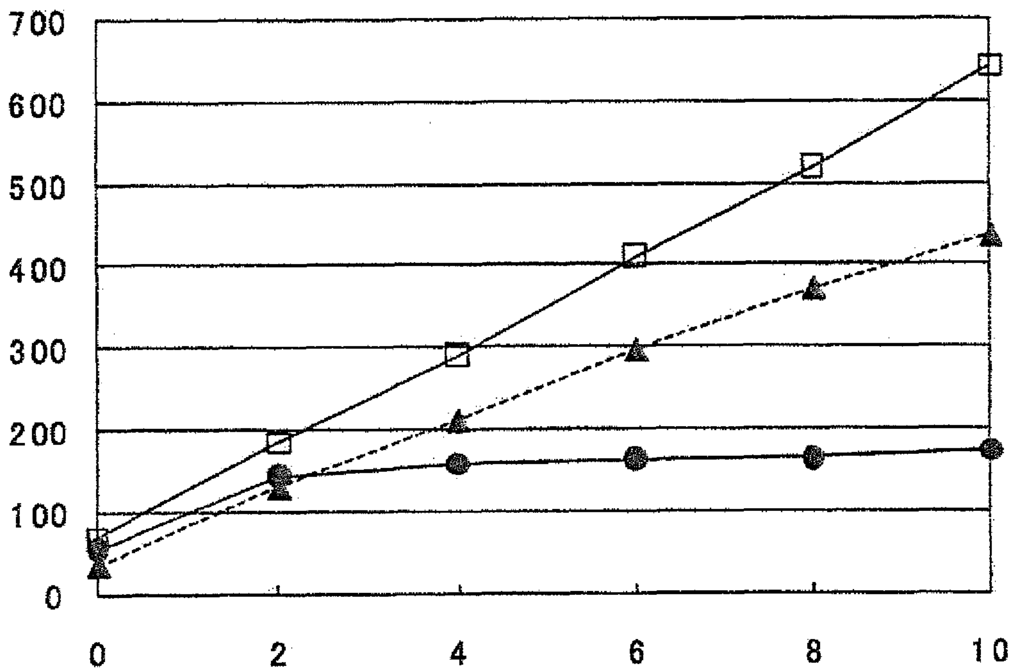

[Figure 3]

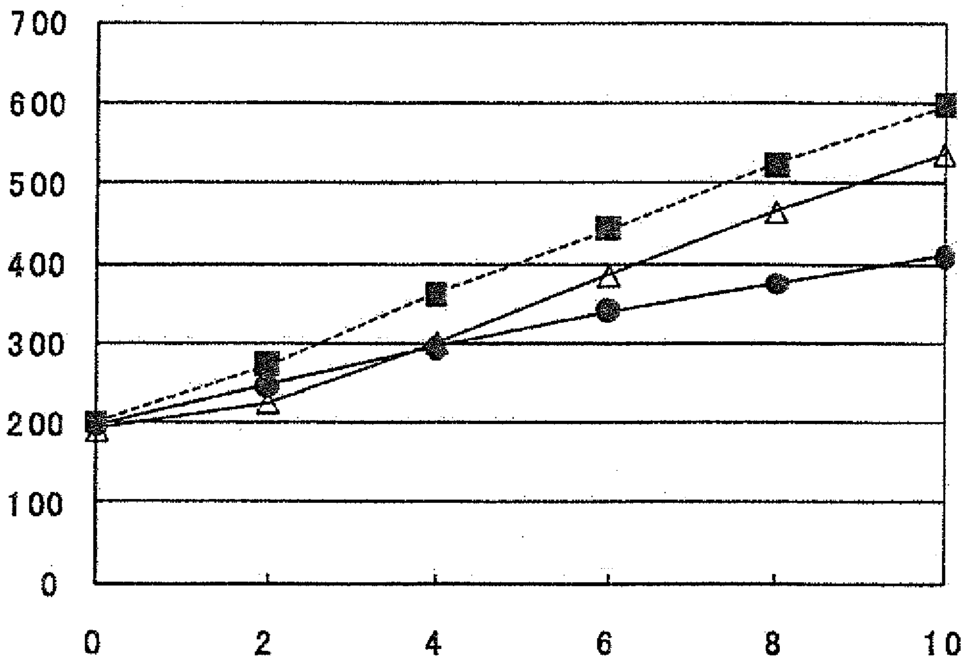


[Figure 4]

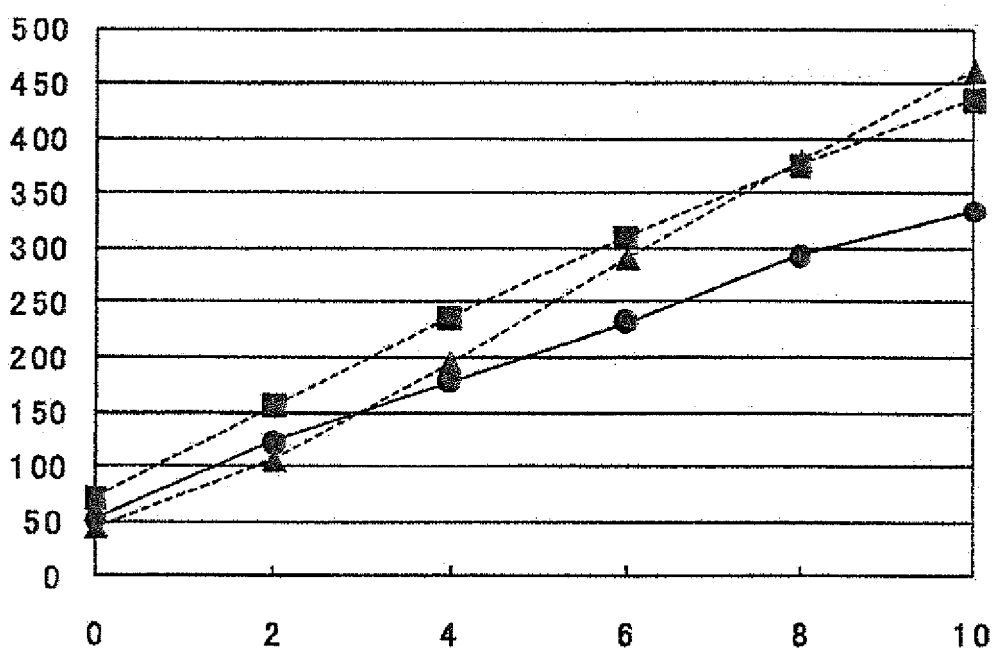

[Figure 5]

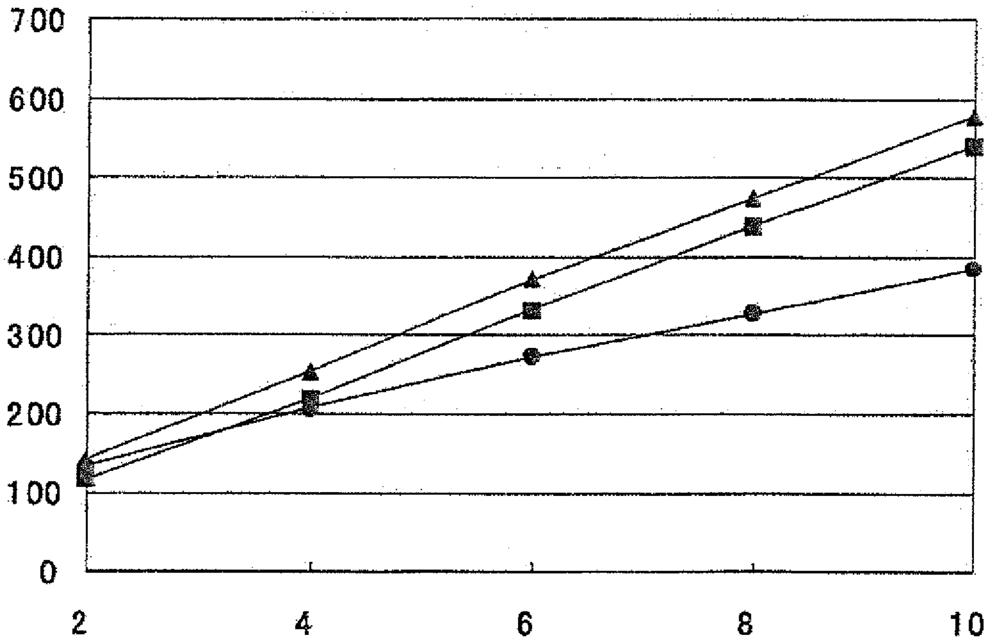

**INTERNATIONAL SEARCH REPORT**

International application No.
PCT/JP2011/068103

A. CLASSIFICATION OF SUBJECT MATTER
*C12Q1/26*(2006.01)i, *C12Q1/28*(2006.01)i, *C12Q1/37*(2006.01)i

According to International Patent Classification (IPC) or to both national classification and IPC

B. FIELDS SEARCHED

Minimum documentation searched (classification system followed by classification symbols)
C12Q1/26, C12Q1/28, C12Q1/37

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/BIOSIS/MEDLINE/WPIDS(STN), JSTPlus/JMEDPlus/JST7580(JDreamII), Science Direct, Wiley InterScience

C. DOCUMENTS CONSIDERED TO BE RELEVANT

| Category* | Citation of document, with indication, where appropriate, of the relevant passages | Relevant to claim No. |
|---|---|---|
| A | Kozo HIROKAWA, "Development of novel enzymes applied to clinical diagnosis for diabetes", BIO INDUSTRY, 12 January 2009 (12.01.2009), vol.26, no.1, pages 73 to 80 | 1-15 |
| A | HIROKAWA, K., et al., An enzymatic method for the determination of hemoglobin$A_{1C}$., Biotechnol. Lett., 2005.07, vol.27, no.14, p.963-968 | 1-15 |
| A | THORMANN, J., Contact dermatitis to a new fungicide, 2-n-octyl-4-isothiazolin-3-one., Contact Dermatitis, 1982.05, vol.8, no.3, p.204 | 1-15 |
| A | CHEW, A.L., et al., 1,2-Benzisothiazolin-3-one (Proxel): irritant or allergen? A clinical study and literature review., Contact Dermatitis, 1997.03, vol.36, no.3, p.131-136 | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 September, 2011 (08.09.11) | 20 September, 2011 (20.09.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3010696 A
- WO 200310701 A
- JP 2007147630 A
- JP 2000210100 A
- WO 200504985 A